(19) Europäisches Patentamt
European Patent Office
Office européen des brevets

(11) EP 3 222 267 A1

(12) **EUROPEAN PATENT APPLICATION**

(43) Date of publication:
**27.09.2017 Bulletin 2017/39**

(21) Application number: **16161829.3**

(22) Date of filing: **23.03.2016**

(51) Int Cl.:
*A61K 8/35* (2006.01) *A61K 8/36* (2006.01)
*A61K 8/41* (2006.01) *A61K 8/42* (2006.01)
*A61K 8/44* (2006.01) *A61Q 5/06* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **The Procter & Gamble Company**
**Cincinnati, OH 45202 (US)**

(72) Inventors:
- **MARSH, Jennifer**
**Cincinnati, OH Ohio 45202 (US)**
- **GALAZKA, Sebastian**
**Cincinnati, OH Ohio 45202 (US)**
- **AGOSTINO, Elizabeth**
**Cincinnati, OH Ohio 45202 (US)**
- **LANE, Brandon**
**Cincinnati, OH Ohio 45202 (US)**
- **STEPHENS, Tracy**
**Cincinnati, OH Ohio 45202 (US)**
- **CONA, M Martha**
**Cincinnati, OH Ohio 45202 (US)**

(74) Representative: **Sauvaître, Thibault Bruno**
**Procter & Gamble Service GmbH**
**Patent Department**
**PO Box 14**
**Frankfurter Straße 145**
**61476 Kronberg im Taunus (DE)**

# (54) METHOD FOR TREATING AND COLORING HAIR COMPRISING UN-PIGMENTED HAIR

(57) A method for treating and coloring hair comprising un-pigmented hair comprises:
(a) Providing a first composition comprising one or more heavy metal ion sequestrants;
(b) Applying the first composition to the hair;
(c) Providing a second composition comprising one or more weak acids having a pKa from 2.5 to 5;
(d) Applying the second composition to the hair;
(e) Providing a third composition wherein the third composition comprises one or more direct dyes which are selected from the group consisting of nitro dye, disperse dye, cationic dye, acid dye, basic dye, neutral azo dye and mixtures thereof, wherein the third composition is able to decrease the reflectance of un-pigmented hair in a wavelength range from 530 nm to 670 nm; and
(f) Applying the third composition to the hair.

EP 3 222 267 A1

## Description

FIELD OF THE INVENTION

A method for treating and coloring hair comprising un-pigmented hair, preferably gray or white hair, is provided and comprises providing a first composition comprising one or more heavy metal ion sequestrants; applying the first composition to the hair; providing a second composition comprising one or more weak acids having a pKa from 2.5 to 5; applying the second composition to the hair; providing a third composition wherein the third composition comprises one or more direct dyes wherein the third composition is able to decrease the reflectance of unpigmented hair in a wavelength range from 530 nm to 670 nm; and applying the third composition to the hair. A first, second and third compositions are also provided.

Also, a kit for treating un-pigmented hair is provided and comprises a first, a second and a third compositions packaged in different containers or in a same container in different compartments.

BACKGROUND OF THE INVENTION

Hair coloring or dyeing involves the application of one or more hair dyes onto hair which results in the coloration of hair fibers. The total head of hair color may be changed subtly or dramatically, the root growth colored to match the remaining head of hair, effects introduced such as glitter, hair strand effects or other sectional effects, or the same color "freshened up" to combat fade and/or wash-out.

It is known to dye keratinous fibres, and in particular human hair comprising unpigmented hair, with dyeing compositions containing oxidation dye precursors. Oxidation dye precursors, or oxidation bases, are colourless or slightly coloured compounds which, when mixed with oxidizing products at the time of use, are able, by a process of oxidative condensation, to give rise to coloured compounds and dyes. It is also known that it is possible to vary the shades obtained with these oxidation bases by combining them with couplers or dye modifiers.

Human un-pigmented hair, gray or white hair, yellows naturally with age, and with the use of yellow-colored shampoos for instance. In the particular case of the dyeing of unpigmented hair in order to remedy the yellowing thereof, while leaving it with a slight silvery or steely glint, it is possible to employ several types of products.

First of all, there are shampoos based on direct dyes which are present in small quantities. Direct dyes are employed without an oxidizing agent and are capable of modifying the natural coloring of the hair more or less strongly. However, although these shampoos give an immediate effect, they have the drawback of giving a result which disappears between two shampooings.

There are also dye products which are presented in the form of a foam or a solution containing direct dyes which are applied to the hair for short waiting times. However, such dye products impart selective colorations to the hair; in other words, they give different shades on sensitized or nonsensitized hair. Moreover, these dye products have inadequate covering power of the hair and disappear rapidly after several shampooings.

In that respect, compositions for dyeing of human hair which have anti-yellow effect for gray hair and comprise at least one direct-acting hair dyestuff have been provided, see for instance European Patent Application EP 0 834 303 A2.

Hair dye using a natural indigo plant for preventing white hair from turning yellowish and of dyeing hair with natural tone while hiding white hair has been provided, see for instance US Patent US 6,849,096 B2.

In general, when coloring un-pigmented hair with direct dyes having a blue or violet shade to remedy the yellowing, the color of un-pigmented hair becomes too blue or violet than it is needed, without embracing natural un-pigmented hair, i.e. glowing, glossing and bright gray hair.

Hence, there is a need to provide a method which can lead to natural gray shades which remedy the yellowing of human un-pigmented hair and which, moreover, can provide other benefits such as providing brilliant and glowing un-pigmented hair, improving the luster and/or the light reflectance of un-pigmented, gray or white hair.

SUMMARY OF THE INVENTION

A method for treating and coloring hair, preferably human hair, comprising un-pigmented hair, preferably gray or white hair, is provided and comprises:

(a) Providing a first composition comprising one or more heavy metal ion sequestrants;
(b) Applying the first composition to the hair;
(c) Providing a second composition comprising one or more weak acids having a pKa from 2.5 to 5, preferably from 2.5 to 4.5, more preferably from 2.5 to 3.5;
(d) Applying the second composition to the hair;
(e) Providing a third composition wherein the third composition comprises one or more direct dyes which are selected

from the group consisting of a nitro dye, a disperse dye, a cationic dye, an acid dye, a basic dye, a neutral azo dye and mixtures thereof, wherein the third composition is able to decrease the reflectance of un-pigmented hair in a wavelength range from 530 nm to 670 nm; and

(f) Applying the third composition to the hair.

The steps (a)-(f) of the method may be performed in that order. Alternatively, the steps (c)-(d) may be performed before the steps (a)-(b).

The second composition may have a pH from 2 to 6, preferably from 2.5 to 6, more preferably from 2.5 to 4.

Each of the first, second and third compositions may be applied to the hair for a time period ranging from 5 min to 30 min, preferably from 5 min to 15 min, more preferably from 10 to 15 min.

Each of the first, second and third compositions may be applied to the hair with a brush and bowl applicator or a container to which a nozzle or a separate applicator device such as a comb or a brush is attached.

A kit for treating un-pigmented hair, preferably gray or white hair, is provided and comprises a first, a second and a third compositions packaged in different containers or in a same container in different compartments; wherein the first composition comprises one or more heavy metal ion sequestrants as defined hereinafter; wherein the second composition comprises one or more weak acids having a pKa from 3 to 5 as defined hereinafter; and wherein the third composition comprises one or more direct dyes as hereinafter.

The kit may further comprise a brush and bowl applicator.

Use of a first, a second and a third compositions as defined hereinafter for improving the luster and/or the light reflectance of un-pigmented hair.

Use of the first composition for purifying un-pigmented hair. Use of the second composition glossing and glowing un-pigmented hair. Use of the third for brightening or increasing the vibrancy of un-pigmented hair.

BRIEF DESCRIPTION OF THE DRAWINGS

While the specification concludes with claims particularly pointing out and distinctly claiming the present invention, it is believed that the same will be better understood from the following description read in conjunction with the accompanying drawings in which:

Fig. 1A is an image of untreated human hair fibers obtained from a scanning electron microscope;

Fig. 1B is an image of human hair fibers according to the method of the present invention, the image being obtained from a scanning electron microscope;

Fig. 2A is an image of untreated human hair fibers obtained from an optical microscope;

Fig. 2B is an image of human hair fibers according to the method of the present invention, the image being obtained from an optical microscope;

Fig. 3 shows the color reflectance of untreated hair strands (M0), and the color reflectance of hair strands only treated respectively with the first composition (M1), or with the second composition (M2), or with the third composition (M3A), or with the respective combination of different treatments (M13A, M23A, M4); and

Fig. 4 shows the color reflectance of untreated hair strands (M0), and the color reflectance of hair strands only treated respectively with the first composition (M1), or with the second composition (M2), or with the third composition (M3B), or with the respective combination of different treatments (M13B, M23B, M5).

DETAILED DESCRIPTION OF THE INVENTION

## Definitions of terms

In this document, including in all embodiments of all aspects of the present invention, the following definitions apply unless specifically stated otherwise.

All percentages are by weight (w/w) of the first composition and/or the second composition and/or the third composition, unless otherwise specified. All ratios or percentages are weight ratios or weight percentages unless specifically stated otherwise."% wt." means percentage by weight. References to "parts" e.g. a mixture of 1 part X and 3 parts Y, is a ratio by weight. When more than one composition are used during a treatment, the total weight to be considered is the total weight of all the compositions applied on the hair simultaneously (i.e. the weight found "on head"), unless otherwise specified.

"QSP" means sufficient quantity for 100% or for 100g. "+/-" indicates the standard deviation. All ranges are inclusive and combinable. The number of significant digits conveys neither a limitation on the indicated amount nor on the accuracy of the measurement. All numerical amounts are understood to be modified by the word "about".

All measurements are understood to be made at 20°C and at ambient conditions, where "ambient conditions"

means at 1 atmosphere (atm) of pressure and at 65% relative humidity, unless otherwise stated. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International.

Herein "min" means "minute" or "minutes". Herein "mol" means mole. Herein "g" following a number means "gram" or "grams". "Ex." means "example". All amounts as they pertain to listed ingredients are based on the active level ("solids") and do not include carriers or by-products that may be included in commercially available materials.

Herein, "comprising" means that other steps and other ingredients can be in addition. "Comprising" encompasses the terms "consisting of" and "consisting essentially of'. The compositions, methods, uses, and kits of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient (e.g. heavy metal ion sequestrant, weak acid, direct dye, etc) or mixtures thereof described hereinafter can account for up to 100% (or 100%) of the total amount of the ingredient(s) in the first composition and/or the second composition and/or the third composition.

The term "substantially free of" as used herein means less than 1%, less than 0.8%, less than 0.5%, less than 0.3%, or less than an immaterial amount of by total weight of the composition.

The term "hair" as used herein means mammalian hair including scalp hair, facial hair and body hair, more preferably hair on the human head and scalp. Hair comprises hair fibers. "Hair shaft" means an individual hair strand and may be used interchangeably with the term "hair." As used herein the term "hair" to be treated may be "living" i.e. on a living body or may be "non-living" i.e. in a wig, hairpiece or other aggregation of non-living keratinous fibers. Mammalian, preferably human hair is preferred.

The term "cosmetically acceptable" as used herein means that the compositions, or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

The term "direct dye" as used herein is a dye than can express color without any aid of other substances. The term "direct dye" encompasses the dyes other than oxidative dyes which first develop color when oxidized by an oxidizing agent such as hydrogen peroxide. Examples of direct dyes include nitro dyes, basic dyes, cationic dye, acid dye, neutral azo dye and disperse dyes.

The term "separately packaged" as used herein means any form of packaging that prevents a first composition from coming into physical contact, or admixing, with a second or a third composition. "Separately packaged" may mean that the individual first, second and third compositions are packaged in separate containers, or alternatively in a single container partitioned such that the first, second and third compositions are not in physical contact.

The term "kit" as used herein means a packaging unit comprising a plurality of components i.e. a kit of parts. An example of a kit is, for example, a first composition, a separately packaged second composition and a separately packaged third composition. Another kit may comprise application instructions comprising a method and a first, second and third compositions.

**Method for treating and coloring hair comprising un-pigmented hair**

A method for treating and coloring hair, preferably human hair, comprising un-pigmented hair, preferably gray or white hair, is provided. With age, hair turns more and more unpigmented. The hair may comprise from 25% to 100%, or from 50% to 100%, or from 75% to 100%, or from 80% to 100%, or from 90% to 100%, or from 95% to 100% of un-pigmented hair by total weight of hair.

Over the time, as hair turns un-pigmented, un-pigmented hair yellows. It has been found a new methodology to remedy the yellowing of un-pigmented hair while improving surprisingly the gray or white shade of un-pigmented hair, namely the glowing, the luster and/or the light reflectance of un-pigmented hair.

The method for treating and coloring hair, preferably human hair, comprising unpigmented hair comprises providing a first composition. The first composition comprises one or more heavy metal ion sequestrants. The method comprises applying the first composition to the hair.

Un-pigmented hair lacks of melanin. Un-pigmented hair is prone to be photodamaged by the ultraviolet light. The oxidation of un-pigmented hair, especially the oxidation of the lipids and proteins comprised in un-pigmented hair by the ultraviolet light is catalyzed by heavy metal ions such as copper, zinc, iron or manganese ions. Impurities may result of the ultraviolet oxidation of tryptophan-containing proteins into especially, 3-hydroxykynurenine, which has a yellow coloration.

By heavy metal ion sequestrant it is meant herein components which act to sequester (chelate or scavenge) heavy metal ions. The one or more heavy metal ion sequestrants may also have calcium and magnesium chelation capacity. However, preferentially, the one or more heavy metal ion sequestrants show selectivity to binding heavy metal ions such as copper, iron, zinc, and manganese ions. The one or more heavy metal ion sequestrants are valuable in the first composition as herein described for the delivery of controlled oxidizing action as well as for the provision of good storage stability of the hair coloring products. Hence, the one or more heavy metal ion sequestrants can help to prevent hair to be photo-damaged by the ultraviolet light.

Levels of copper metal have been quantified in untreated and treated human hair strands using the inductively coupled plasma atomic spectroscopy (see experimental part).

When the human hair fibers are treated with the first composition comprising a heavy metal ion sequestrant, the level of copper remaining on the human hair fibers has been relatively reduced compared to untreated human hair fibers.

Also, it has been observed that after un-pigmented hair has been treated with the first composition, un-pigmented hair appears purified from heavy metal ions. The treated hair already appears slightly less yellow than untreated hair.

The one or more heavy metal ion sequestrants may be selected from the group consisting of nitrilotriacetic acid, ethylenediaminotetracetic acid, ethylenetriamine pentaacetic acid, ethylenediamine-N,N'-disuccinic acid (EDDS), ethylenediamine-N,N'-diglutaric acid (EDDG), 2-hydroxypropylenediamine-N,N'-disuccinic acid (HPDDS), glycinamide-N,N'-disuccinic acid (GADS), ethylenediamine-N,N'-bis(ortho-hydroxyphenyl acetic acid) (EDDHA), N,N'-bis(2-hydroxybenzyl)ethylenediamine-N,N'-diacetic acid (HBED), aminotri-(methylenephosphonic acid), ethylene-diaminotetra-(methylenephosphonic acid) (EDTMP), diethylene-triamine-penta-(methylenephosphonic acid) (DTPMP), salts thereof, and mixtures thereof.

The one or more heavy metal ion sequestrants may be preferably selected from the group consisting of ethylenediamine-N,N'-disuccinic acid (EDDS), salts thereof, and mixtures thereof.

The one or more heavy metal ion sequestrants may be used in their alkali metal, alkaline earth metal, ammonium, or substituted ammonium salts thereof, or mixtures thereof, such as sodium, calcium, magnesium or ammonium salts, for instance.

Other various heavy metal ion sequestrants may also be contemplated, including the amino phosphonates, available as Dequest (RTM) from Monsanto, the nitriloacetates, the hydroxyethyl-ethylene triamines and the like which are known for such use. Suitable heavy metal ion sequestrants for use herein may include organic phosphonates, such as the amino alkylene poly (alkylene phosphonates), alkali metal ethane 1-hydroxy disphosphonates and nitrilo trimethylene phosphonates.

Preferred among the above species may be diethylene triamine penta(methylene phosphonate), ethylene diamine tri(methylene phosphonate) hexamethylene diamine tetra (methylene phosphonate) and hydroxy-ethylene 1,1 diphosphonate.

Other suitable heavy metal ion sequestrants for use herein may be iminodiacetic acid derivatives such as 2-hydroxyethyl diacetic acid or glyceryl iminodiacetic acid, described in EP-A-317,542 and EP-A-399,133. The iminodiacetic acid-N-2-hydroxypropyl sulfonic acid and aspartic acid N-carboxymethyl N-2-hydroxypropyl-3-sulfonic acid sequestrants described in EP-A-516,102 are also suitable herein. The β-alanine-N,N'-diacetic acid, aspartic acid-N,N'-diacetic acid, aspartic acid-N-monoacetic acid and iminodisuccinic acid sequestrants described in EP-A-509,382 are also suitable.

The total amount of the one or more heavy metal ion sequestrants may be from 0.005% to 20%, preferably from 0.01% to 10%, more preferably from 0.05% to 2% by total weight of the first composition.

The first composition may be used to purify un-pigmented hair from heavy metal ions responsible of premature oxidation damage.

The method for treating and coloring hair, preferably human hair, comprising unpigmented hair comprises providing a second composition. The second composition comprises one or more weak acids having a pKa from 2.5 to 5, preferably from 2.5 to 4.5, more preferably from 2.5 to 3.5. The method comprises applying the second composition to the hair.

Human hair typically comprises fatty acid deposits and/or calcium carbonate deposits. Fatty acid deposits may be such as calcium or magnesium fatty acid deposits, e.g. calcium palmitate or calcium stearate. Other deposits such as magnesium/silicone, and magnesium/aluminum deposits have been characterized by Energy dispersive X-ray spectroscopy. At a relatively low pH, the heavy metal ion sequestrants cannot be used to bind with a fatty acid salt.

The second composition comprises one or more weak acids having a pKa from 2.5 to 5. The one or more weak acids of the second composition may preferably be able to bind with a fatty acid salt. When applying to the hair the

second composition comprising one or more weak acids having a pKa from 2.5 to 5, the second composition can help to dissolve calcium or magnesium fatty acid deposits and/or calcium carbonate deposits.

The second composition may have a pH from 2 to 6, preferably from 2.5 to 6, more preferably from 2.5 to 4. The one or more weak acids may be used to adjust the pH of the second composition. In the case where the concentration of the selected one or more weak acids is not enough to reached the selected pH, other organic or inorganic acids may as well be used to adjust the pH of the second composition to the required value. A pH from 2 to 6 for the second composition can help to dissolve calcium carbonate deposits found on the hair.

The one or more weak acids of the second composition may be preferably selected from the group consisting of succinic acid, maleic acid, malonic acid, glycolic acid, acotinic acid, 2-picolinic acid, L-tartric acid, D-tartric acid, citric acid, isocitric acid, ascorbic acid, lactic acid, benzoic acid, oxalic acid, acetic acid, formic acid, propionic acid and mixtures thereof.

The one or more weak acids of the second composition may be more preferably selected from the group consisting of succinic acid, maleic acid, malonic acid, glycolic acid, acotinic acid, 2-picolinic acid, L-tartric acid, citric acid, isocitric acid, and mixtures thereof. Hence, the one or more weak acids of the second composition can be able to dissolve calcium or magnesium fatty acid deposits and/or calcium carbonate deposits.

The total amount of the one or more weak acids may be from 0.5% to 20%, preferably from 0.1% to 10%, more preferably from 1% to 5% by total weight of the second composition. Fig. 1A is an image of untreated human hair fibers obtained from a scanning electron microscope. Significant white deposits 11 have been seen on an untreated human hair fiber 10 before any application of each of the first or second composition.

Fig. 1B is an image of human hair fibers according to the method of the present invention, the image being obtained from a scanning electron microscope. Relatively lower levels of white deposits 11 in treated human hair fibers 10 according to the method of the present invention have been observed compared to the levels of white deposits for untreated human hair fibers. Hence, the second composition may be used to improve the gloss of un-pigmented hair.

The method for treating and coloring hair, preferably human hair, comprising unpigmented hair comprises providing a third composition. The third composition comprises one or more direct dyes which are selected from the group consisting of a nitro dye, a disperse dye, a cationic dye, an acid dye, a basic dye, a neutral azo dye and mixtures thereof, wherein the third composition is able to decrease the reflectance of un-pigmented hair in a wavelength range from 530 nm to 670 nm.

The one or more direct dyes may be selected from the group consisting of one or more nitro dyes to provide either a violet or a blue color. The one or more direct dyes may be selected from the group consisting of one or more basic dyes to provide either a violet or a blue color. The one or more direct dyes may be selected from the group consisting of one or more disperse dyes to provide either a violet color or a blue color.

The one or more direct dyes may be selected from the group consisting of Acid dyes such as Acid Yellow 1, Acid Orange 3, Acid Black 1, Acid Black 52, Acid Orange 7, Acid Red 33, Acid Yellow 23, Acid Blue 9, Acid Violet 43, Acid Blue 16, Acid Blue 62, Acid Blue 25, Acid Red 4, Acid Red 18, Acid Red 52, Acid Red 92, Acid Green 25, Basic Dyes such as Basic Brown 17, Basic Red 118, Basic Orange 69, Basic Red 76, Basic Brown 16, Basic Yellow 57, Basic Violet 14, Basic Violet 2, Basic Blue 7, Basic Blue 26, Basic Red 2, Basic Blue 99, Basic Yellow 29, Basic Red 51, Basic Orange 31, Basic Yellow 87, Basic Blue 124, 4-(3-(4-amino-9,10-dioxo-9,10-dihydroanthracen-1-ylamino)propyl)-4-methylmorpholin-4-ium-methylsulfate, (E)-1-(2-(4-(4,5-dimethylthiazol-2-yl)diazenyl)phenyl)(ethyl)amino)ethyl)-3-methyl-1H-imidazol-3-ium chloride, (E)-4-(2-(4-(dimethylamino)phenyl)diazenyl)-1-methyl-1H-imidazol-3-ium-3-yl)butane-1-sulfonate, (E)-4-(4-(2-methyl-2-phenylhydrazono)methyl)pyridinium-1-yl)butane-1-sulfonate, N,N-dimethyl-3-(4-(methylamino)-9,10-dioxo-4a,9,9a,10-tetrahydroanthracen-1-ylamino)-N-propylpropan-1-aminium bromide, Disperse Dyes such as Disperse Red 17, Disperse Violet 1, Disperse Red 15, Disperse Black 9, Disperse Blue 3, Disperse Blue 23, Disperse Blue 377, Nitro Dyes such as 1-(2-(4-nitrophenylamino)ethyl)urea, 2-(4-methyl-2-nitrophenylamino)ethanol, 4-nitrobenzene-1,2-diamine, 2-nitrobenzene-1,4-diamine, Picramic acid, 2-hydroxyethyl-picramic acid HC Red No. 13, 2,2'-(2-nitro-1,4-phenylene)bis(azanediyl)diethanol, HC Yellow No. 5, HC Red No. 7, HC Blue No. 2, HC Yellow No. 4, HC Yellow No. 2, HC Orange No. 1, HC Red No. 1, 2-(4-amino-2-chloro-5-nitrophenylamino)ethanol, HC Red No. 3, 4-amino-3-nitrophenol, 4-(2-hydroxyethylamino)-3-nitrophenol, 2-amino-3-nitrophenol, 2-(3-(methylamino)-4-nitrophenoxy)ethanol, 3-(3-amino-4-nitrophenyl)propane-1,2-diol, HC Yellow No. 11, HC Violet No. 1, HC Orange No. 2, HC Orange No. 3, HC Yellow No. 9, HC Red No. 10, HC Red No. 11, 2-(2-hydroxyethylamino)-4,6-dinitrophenol, HC Blue No. 11, HC Blue No. 12, HC Yellow No. 6, HC Yellow No. 12, HC Blue No. 10, HC Yellow No. 7, HC Yellow No. 10, HC Blue No. 9, 2-chloro-6-(ethylamino)-4-nitrophenol, 6-nitropyridine-2,5-diamine, HC Violet No. 2, 2-amino-6-chloro-4-nitrophenol, 4-(3-hydroxypropylamino)-3-nitrophenol, HC Yellow No. 13, 6-nitro-1,2,3,4-tetrahydroquinoxaline, HC Red No. 14, HC Yellow No. 15, HC Yellow No. 14, N2-methyl-6-nitropyridine-2,5-diamine, N1-allyl-2-nitrobenzene-1,4-diamine, HC Red No. 8, HC Green No.1, HC Blue No. 7, HC Blue No. 14, HC Blue No. 15, HC Blue No. 16, HC Blue No. 17, and Natural dyes such as Annato, Anthocyanin, Beetroot, Carotene, Capsanthin, Lycopene, Chlorophyll, Henna, Indigo, Cochineal, and mixtures thereof.

The one or more direct dyes may be preferably selected from the group consisting of Basic Brown 17, Basic

Red 118, Basic Orange 69, Basic Red 76, Basic Brown 16, Basic Yellow 57, Basic Violet 14, Basic Blue 7, Basic Blue 26, Basic Red 2, Basic Blue 99, Basic Yellow 29, Basic Red 51, Basic Orange 31, Basic Yellow 87, Basic Blue 124, Disperse Red 17, Disperse Violet 1, Disperse Red 15, Disperse Black 9, Disperse Blue 3, Disperse Blue 23, Disperse Blue 377, HC Red No. 13, HC Yellow No. 5, HC Red No. 7, HC Blue No. 2, HC Yellow No. 4, HC Yellow No. 2, HC Orange No. 1, HC Red No. 1, HC Red No. 3, HC Yellow No. 11, HC Violet No. 1, HC Orange No. 2, HC Orange No. 3, HC Yellow No. 9, HC Red No. 10, HC Red No. 11, HC Blue No. 12, HC Yellow No. 6, HC Yellow No. 12, HC Blue No. 10, HC Yellow No. 7, HC Yellow No. 10, HC Blue No. 9, HC Violet No. 2, HC Yellow No. 13, HC Red No. 14, HC Yellow No. 15, HC Yellow No. 14, HC Red No. 8, HC Green No.1, HC Blue No. 14, HC Blue No. 16, and mixtures thereof.

The one or more direct dyes may be more preferably selected from the group consisting of Basic Red 118, Basic Red 76, Basic Violet 14, Basic Blue 7, Basic Blue 26, Basic Red 2, Basic Blue 99, Basic Red 51, Basic Blue 124, Disperse Red 17, Disperse Violet 1, Disperse Red 15, Disperse Blue 3, Disperse Blue 23, Disperse Blue 377, HC Red No. 13, HC Red No. 7, HC Blue No. 2, HC Red No. 1, HC Red No. 3, HC Violet No. 1, HC Red No. 10, HC Red No. 11, HC Blue No. 12, HC Blue No. 10, HC Blue No. 9, HC Violet No. 2, HC Red No. 14, HC Red No. 8, HC Blue No. 14, HC Blue No. 16, and mixtures thereof.

After applying to the hair the first and the second composition, un-pigmented hair may still comprise some yellowish shades. It should be noted that the third composition when used for treating un-pigmented hair, such as gray or white hair, for example, can help to remedy the undesirable and remaining yellowish shades of un-pigmented hair.

The reflectance of the hair when irradiated with visible light in the range of wavelengths from 400 to 700 nanometers has been measured for several method examples as shown in Fig. 3 and in Fig. 4. If we measure the reflectance of the hair when irradiated with visible light in the range of wavelengths from 400 to 700 nanometers, and comparing the reflectance curves as a function of length wave, the reflectance curve corresponding to the hair only treated with the third composition of the invention (see Method Example M3A or M3B), in a range of wavelengths from 530 to 670 nanometers, is lower than the reflectance curve corresponding to the untreated hair (see Reference M0).

This means that, in the range of wavelengths from 530 to 670 nanometers, and preferably from 540 to 660 nanometers, there are at least a range where the reflectance curve corresponding to the treated hair with only the third composition is lower than the reflectance curve corresponding to the untreated hair. The term "lower" as used herein means a gap of at least 0.05% reflectance, preferably at least 0.1% up to 10%, more preferably at least 0.1% up to 5%, even more preferably up to 2%.

Indeed, when un-pigmented hair has been treated with only the third composition, the third composition can help to remedy the yellowing of the hair. Un-pigmented hair gets a violet (corresponding to the curve M3A in Fig. 3) or a blue shade (corresponding to the curve M3B in Fig. 4). Hence, the third composition is able to decrease the reflectance of un-pigmented hair in a wavelength range from 530 nm to 670 nm. At the wavelength range from 530 nm to 670 nm, the selected one or more direct dyes can absorb the yellow-red light spectrum. Hence, the reflectance at the wavelength range from 530 nm to 670 nm decreases. Consequently, un-pigmented hair appears relatively less yellow as compared to untreated un-pigmented hair.

Preferably, the wavelength where the difference is the maximum between the reflectance curve of the hair only treated with the third composition and the reflectance curve of the untreated hair is in the range of wavelength from 570 to 640 nanometers, and preferably in the range of wavelengths from 580 to 600 nanometers.

The method may be able to decrease the reflectance of unpigmented hair in a wavelength range from 530 nm to 670 nm, preferably from 540 nm to 670 nm, more preferably from 560 nm to 650 nm, even more preferably from 570 nm to 630 nm, further than when only applying the third composition to hair.

It has been surprisingly found that when the first and second compositions have been applied in any order to the hair, following by the application of the third composition as set out hereinabove, a further decrease of the reflectance of unpigmented hair in a wavelength range from 530 nm to 670 nm could be obtained.

As shown in Fig. 3, the reflectance curve corresponding to the hair treated according to the method of the present invention, i.e. with the first, second and third compositions (see Method Example M4), in a range of wavelengths from 530 to 670 nanometers, is lower than the reflectance curve corresponding to the hair treated only by the same third composition (see Reference M3A).

Similarly, as shown in Fig. 4, the reflectance curve corresponding to the hair treated according to the method of the present invention, i.e. with the first, second and third compositions (see Method Example M5), in a range of wavelengths from 530 to 670 nanometers, is lower than the reflectance curve corresponding to the hair treated only by the same third composition (see Reference M3B).

This means that, in the range of wavelengths from 530 to 670 nanometers, and preferably from 540 to 670 nanometers, more preferably from 560 nm to 650 nm, even more preferably from 570 nm to 630 nm, there are at least a range where the reflectance curve corresponding to the treated hair according to the method of the present invention is lower than the reflectance curve corresponding to the hair treated with only the third composition.

The reflectance curve corresponding to the hair treated according to the method of the present invention and the reflectance curve corresponding to the hair treated with only the third composition may be separated by a gap. The

gap between the reflectance curve corresponding to the hair treated according to the method of the present invention and the reflectance curve corresponding to the hair treated with only the third composition may be of at least 0.05% reflectance, preferably at least from 0.1% to 5% reflectance, preferably from 0.1% to 2% reflectance.

The steps (a)-(f) of the method may be performed in that order. Alternatively, the steps (c)-(d) may be performed before the steps (a)-(b).

Without wishing to be bound by theory, it is believed that when un-pigmented hair has been treated by the first and second compositions in any order, the deposits mainly responsible of the yellowing of un-pigmented hair, i.e. heavy metal ions which catalyzed UV oxidation of unpigmented hair, calcium or magnesium fatty acid deposits, calcium carbonate deposits, have been removed or treated such un-pigmented hair gets relatively pure, glossing and glowing. At this stage, only a residual yellowish shade remaining on un-pigmented hair needs to be treated by the third composition. Hence, the method of the present invention can help to improve the decrease of the reflectance of un-pigmented hair since all the main deposits responsible to the yellowing of un-pigmented hair have been removed or treated before applying the third composition.

Also, the luster of un-pigmented hair treated according to the method of the present invention has been measured and compared to untreated hair (see experimental part). Human unpigmented hair fibers treated according to the method of Example M4 versus untreated human un-pigmented hair fibers showed a significant increase of luster, and thus of the shine. On pigmented hair (i.e. non-gray or non-white hair, comprising melanin), the visible light is absorbed by the melanin. Unlike pigmented hair, the visible light on un-pigmented hair is reflected both from the surface and internally from the back side of un-pigmented hair. The removal of surface deposits due to the application of the first and second compositions prior to the third composition has been translated into a relative increase of the luster, i.e. the shine. The method of the present invention can help to improve the brightness of un-pigmented hair. The method of the present invention can help to prevent the tone of un-pigmented hair to be flat, dirty and/or dull.

The total amount of the one or more direct dyes of the third composition may be from 0.001% to 5.0%, preferably from 0.003% to 2.5%, more preferably from 0.005% to 1.5%, even more preferably from 0.005% to 1.0%, or from 0.005% to 0.1%, or from 0.005% to 0.05% by total weight of the third composition. Indeed, only low concentrations of the one or more direct dyes can be used to cover the residual yellowish shade since the majority of the deposits responsible of the yellowing of un-pigmented hair has been removed by the first and the second compositions.

The third composition may preferably comprise one or more direct dyes which are selected from the group consisting of nitro dye, disperse dye and mixtures thereof; and the third composition may be applied to hair comprising from 80% to 100%, preferably from 90% to 100%, more preferably from 95% to 100% of un-pigmented hair.

The third composition may be used for the method of the present invention in the case when the hair comprises from 80% to 100%, preferably from 90% to 100%, more preferably from 95% to 100% of un-pigmented hair. The third composition may be applied preferably to hair comprising a relatively high percentage of un-pigmented hair. The third composition can be able to counteract the yellowish discoloration, while at the same time not providing an overall dark or a relatively artificial tone.

The one or more direct dyes may be more preferably selected from the group consisting of Disperse Red 17, Disperse Violet 1, Disperse Red 15, Disperse Blue 3, Disperse Blue 23, Disperse Blue 377, HC Red No. 13, HC Red No. 7, HC Blue No. 2, HC Red No. 1, HC Red No. 3, HC Violet No. 1, HC Red No. 10, HC Red No. 11, HC Blue No. 12, HC Blue No. 10, HC Blue No. 9, HC Violet No. 2, HC Red No. 14, HC Red No. 8, HC Blue No. 14, HC Blue No. 16 and mixtures thereof and the third composition may be applied to hair comprising from 80% to 100%, preferably from 90% to 100%, more preferably from 95% to 100% of un-pigmented hair.

In Fig. 3, such third composition has a violet shade. The third composition of method example M4 comprises a mixture of HC Blue No. 2 and Disperse Violet 1.

The third composition may be intended for individuals with relatively high percentages of un-pigmented hair. In that case, the third composition having very little color can hardly contribute to the overall color. Hence, the third composition such as a subtle violet tone, can help to neutralize the yellowish discoloration in un-pigmented hair, as shown on Fig. 3. In terms of reflectance, the third composition can predominantly reduce the reflectance of yellow to orange wavelengths from 560 to 630 nm.

Alternatively, the third composition may preferably comprise one or more direct dyes which are selected from the group consisting of nitro dye, cationic dye, acid dye, basic dye and mixtures thereof and the third composition may be applied to the hair comprising from 25% to 90%, preferably from 50% to 85%, more preferably from 75% to 85% of un-pigmented hair.

The third composition may be used for the method of the present invention in the case when the hair comprises from 25% to 90%, preferably from 50% to 85%, more preferably from 75% to 85% of un-pigmented hair. The third composition can help to counteract the remaining yellow discoloration, while at the same time taking into account the ratio of un-pigmented hair versus pigmented hair. Indeed, pigmented hair can act as a contrast and can contribute to the overall color.

The third composition may preferably comprise one or more direct dyes which are selected from the group

consisting of of Basic Red 118, Basic Red 76, Basic Violet 14, Basic Blue 7, Basic Blue 26, Basic Red 2, Basic Blue 99, Basic Red 51, Basic Blue 124, Disperse Red 17, Disperse Violet 1, Disperse Red 15, Disperse Blue 3, Disperse Blue 23, Disperse Blue 377, HC Red No. 13, HC Red No. 7, HC Blue No. 2, HC Red No. 1, HC Red No. 3, HC Violet No. 1, HC Red No. 10, HC Red No. 11, HC Blue No. 12, HC Blue No. 10, HC Blue No. 9, HC Violet No. 2, HC Red No. 14, HC Red No. 8, HC Blue No. 14, HC Blue No. 16, and mixtures thereof and the third composition is applied to the hair comprising from 25% to 90%, preferably from 50% to 85%, more preferably from 75% to 85% of un-pigmented hair.

In Fig. 4, such third composition has a blue shade. The third composition of method example M5 comprises a mixture of HC Red No. 3, HC Blue No. 16, HC Blue No. 2 and Basic Red 76.

The third composition may be intended for individuals having hair with mixed pigmented and un-pigmented hair, i.e. having hair comprising from 25% to 90% of un-pigmented hair. As pigmented hair can contribute to the overall color observed on the hair, the shade of the third composition can provide a relatively deeper and cooler blue tone which is efficacious in neutralizing the yellowish discoloration observed on un-pigmented hair, while at the same time enhancing the contrast between un-pigmented and pigmented hair. The color benefit is demonstrated in Fig. 4, where the third composition used in the method of the present invention can be efficacious in reducing the reflectance of the yellow, orange and red wavelengths vs. an untreated hair.

Further treatments

One or more of the steps chosen from the steps (b), (d) and (f) may be followed by treating the hair with a treatment selected from the group consisting of rinsing, shampooing, conditioning and combinations thereof.

The step (b) may be followed by shampooing and conditioning. Shampooing and conditioning after step (b) can help to remove the heavy metals ions from hair and improve the purification of hair, especially un-pigmented hair.

The application of the first composition to the hair may be followed by treating the hair with a treatment selected form the group consisting of rinsing, shampooing, conditioning and combinations thereof. These further treatments steps can help to remove heavy metal ions from un-pigmented hair.

The application of the second composition to the hair may be followed by treating the hair with a treatment selected form the group consisting of rinsing, shampooing, conditioning and combinations thereof. These further treatment steps can help to remove from un-pigmented hair, white deposits (e.g. calcium or magnesium fatty acid deposits, calcium carbonate deposits) bound to the one or more weak acids of the second composition.

The application of the third composition to the hair may be followed by treating the hair with a treatment selected form the group consisting of rinsing, shampooing, conditioning and combinations thereof.

The step (d) and/or the step (f) may be followed by rinsing, shampooing and conditioning. Rinsing, shampooing and conditioning after the step (d) and/or the step (f) can help to improve the glowing and brightness of hair, especially un-pigmented hair.

A suitable shampoo for shampooing after step (b) and/or step (d) and/or step (f) may be Wella Luxe Oil Keratin Protect shampoo. A suitable conditioner for conditioning after step (b) and/or step (d) and/or step (f) may be Wella Luxe Oil Keratin Restore Mask conditioner.

Each of the first, second and third compositions may applied to the hair for a time period ranging from 5 min to 30 min, preferably from 5 min to 15 min, more preferably from 10 to 15 min. Each step can be done subsequently after the previous one from immediately to a time lasting up to one week.

The first composition may be applied to the hair for a time period ranging from 5 min to 30 min, preferably from 5 min to 15 min, more preferably from 10 to 15 min.

The second composition may be applied to the hair for a time period ranging from 5 min to 30 min, preferably from 5 min to 15 min, more preferably from 10 to 15 min.

The third composition may be applied to the hair for a time period ranging from 5 min to 30 min, preferably from 5 min to 15 min, more preferably from 10 to 15 min.

**Optional ingredients for the first, second and third compositions**

The first composition and/or the second composition and/or the third composition according to the present invention may comprise, in addition to the ingredients indicated above, further ingredients in order to further enhance the properties of the first composition and/or the second composition and/or the third composition, as long as these are not excluded by the claims.

Suitable further ingredients may include, but not limited to: conditioning agents, cosmetically acceptable carrier, solvent, surfactants, thickening agents, hydrophobic phase constituents, pigments, antioxidants, preservatives, perfume and mixtures thereof.

Suitable further ingredients referred to above, but not specifically described below, are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific adjuvants to achieve a particular purpose or multipurpose. A few of these ingredients are discussed hereinbelow, whose disclosure is of course non-exhaustive.

Conditioning agent

The first composition and/or the second composition and/or the third composition may comprise one or more conditioning agents. The one or more conditioning agents of the first composition and/or the second composition and/or the third composition may be selected from the group consisting of silicone materials, amino silicones, fatty alcohols, polymeric resins, polyol carboxylic acid esters, cationic polymers, cationic surfactants, insoluble oils and oil derived materials and mixtures thereof. The one or more conditioning agents of the first composition and/or the second composition and/or the third composition may be selected from the group consisting of mineral oils, glycerine, sorbitol and mixtures thereof.

The first composition and/or the second composition and/or the third composition may comprise from 0.05% to 20%, or from 0.1% to 15%, or from 0.2% to 10%, or from 0.2% to 2%, or from 0.5% to 2% of the one or more conditioning agents by total weight of the respective first composition and/or the second composition and/or the third composition. The one or more conditioning agents may be included in a separate pre- and/or post-treatment composition.

Suitable conditioning agents may include, but are not limited to: silicones, aminosilicones, fatty alcohols, polymeric resins, polyol carboxylic acid esters, cationic polymers, cationic surfactants, insoluble oils and oil derived materials and mixtures thereof. Additional conditioning agents may include mineral oils and other oils such as glycerin and sorbitol.

Particularly useful conditioning agents for the first composition and/or the second composition and/or the third composition may be cationic polymers and/or silicones. Cationic polymers may be chosen from those comprising units of at least one amine group chosen from primary, secondary, tertiary and quaternary amine groups that may either form part of the main polymer chain, or be borne by a side substituent that is directly attached to the main polymer chain.

The one or more conditioning agents of the first composition and/or the second composition and/or the third composition may be a silicone. The silicone of the first composition and/or the second composition and/or the third composition may be selected from the group consisting of polyalkylsilioxane oils, linear polydiemthylsiloxane oils containing trimethylsilyl or hydroxydimethylsiloxane end groups, polymethylphenylsiloxane polydimethylphenylsiloxane, polydimethyldiphenylsiloxane oils, silicone resins, organofunctional siloxanes having in their general structure one or a number of organofunctional group(s), the same or different, attached directly to the siloxane chain, and mixtures thereof. Said organofunctional group(s) may be selected from: polyethyleneoxy and/or polypropyleneoxy groups, (per)fluorinated groups, thiol groups, substituted or unsubstituted amino groups, carboxylate groups, hydroxylated groups, alkoxylated groups, quaternium ammonium groups, amphoteric, betain groups and mixtures thereof. The silicone of the first composition and/or the second composition and/or the third composition may be either used as a neat fluid or in the form of a pre-formed emulsion.

Cosmetically acceptable carrier

The first composition and/or the second composition and/or the third composition may comprise a cosmetically acceptable carrier. The cosmetically acceptable carrier of the first composition and/or the second composition and/or the third composition may be an aqueous carrier. The first composition and/or the second composition and/or the third composition may comprise water. Water can provide a hydrophilic phase, which the hydrophilic portions of any other ingredients comprised in the first composition and/or the second composition and/or the third composition can interact with water. Water can also provide a fluid phase meaning that the first composition and/or the second composition and/or the third composition can be in liquid form and therefore easily mixed with other fluid compositions such as an oxidizing composition. The first composition and/or the second composition and/or the third composition may comprise from 50% to 85% water, or from 65% to 75% of water by total weight of the respective first composition and/or the second composition and/or the third composition.

The cosmetically acceptable carrier may be any carrier suitable for formulating the one or more linkers or the one or more electrophile ingredients into the respective first composition and/or the second composition and/or the third composition being suitable for application onto hair. The cosmetically acceptable carrier may be selected from either an aqueous medium or an aqueous-alcoholic medium. When the cosmetically acceptable carrier is an aqueous-alcoholic carrier, the cosmetically acceptable carrier may comprise water and an alcohol. An alcohol can advantageously influence the viscosity of a relatively wide spectrum of ingredients of the first composition and/or the second composition and/or the third composition. The alcohol of the first composition and/or the second composition and/or the third composition may be selected from the group consisting of: ethanol, isopropanol, propanol, and mixtures thereof.

When the cosmetically acceptable carrier is an aqueous carrier, the aqueous carrier may consist essentially of water and may be substantially free of alcohol. The first composition and/or the second composition and/or the third

composition may comprise a safe and effective amount of cosmetically acceptable carrier which is water. The first composition and/or the second composition and/or the third composition may comprise from 0.1% to 99%, or from 1% to 98%, or from 10% to 97%, or from 30% to 95% of water by total weight of the respective first composition and/or the second composition and/or the third composition.

The first composition and/or the second composition and/or the third composition may be substantially free of alcohol, such as volatile alcohols (e.g. ethanol, isopropanol, propanol). When the first composition and/or the second composition and/or the third composition is substantially free of alcohol, the first composition and/or the second composition and/or the third composition can have advantageously a reduced odour. Flammability issues can also be prevented.

The cosmetically acceptable carrier of the first composition and/or the second composition and/or the third composition may be an oily compound. The oily compound may be selected from the group consisting of cyclic silicones and volatile hydrocarbons. Cyclic silicones can be available from Dow Corning. The cyclic silicone may have from at least 3 silicone atoms or from at least 5 silicone atoms but no more than 7 silicone atoms or no more than 6 silicone atoms. The cyclic silicone may conform to the formula:

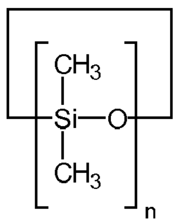

wherein n is from 3 or from 5 but no more than 7 or no more than 6. The cyclic silicone may have a kinematic viscosity of less than 10 cSt at 23°C. A Suitable cyclic silicone for use herein may include Cyclomethicone D5 (commercially available from G.E. Silicones). Alternatively, the first composition and/or the second composition and/or the third composition may be silicone-free.

Volatile hydrocarbons e.g. Isopar can be obtained from ExxonMobil Petroleum and Chemical. The oily compound may be a mineral oil. Trade names for suitable mineral oils include Benol, Blandol, Hydrobrite, Kaydol (Sonneborn LLC Refined Products), Chevron Superla White Oil (Chevron Products Company), Drakeol, Parol (Calumet Penreco LLC), Peneteck (Calumet Penreco LLC), Marcol, and Primol 352 (ExxonMobil Petroleum and Chemical).

Solvent

The first composition and/or the second composition and/or the third composition may further comprise one or more solvents. The one or more solvents may be selected from water, or a mixture of water and at least one organic solvent to dissolve the compounds that would not typically be sufficiently soluble in water.

Suitable organic solvents for the first composition and/or the second composition and/or the third composition may include, but are not limited to: from $C_2$ to $C_4$ lower alkanols (such as ethanol, propanol, isopropanol); aromatic alcohols (such as benzyl alcohol and phenoxyethanol); polyols and polyol ethers (such as carbitols, 2-butoxyethanol, propylene glycol, propylene glycol monomethyl ether, diethylene glycol monoethyl ether, monomethyl ether, hexylene glycol, glycerol, ethoxy glycol, butoxydiglycol, ethoxydiglycerol, dipropyleneglocol, polygylcerol); propylene carbonate; and mixtures thereof.

The one or more solvents of the first composition and/or the second composition and/or the third composition may be selected from the group consisting of water, ethanol, propanol, isopropanol, glycerol, 1,2-propylene glycol, hexylene glycol, ethoxy diglycol, and mixtures thereof.

Typically, the first composition and/or the second composition and/or the third composition may comprise water as a main ingredient, particularly in a total amount ranging from at least 50%, alternatively from at least 60%, alternatively from at least 70%, by total weight of the respective first composition and/or the second composition and/or the third composition. Typically, when present, the first composition and/or the second composition and/or the third composition may comprise a total amount of organic solvents ranging from 1% to 30%, by total weight of the respective first composition and/or the second composition and/or the third composition.

Surfactant

The first composition and/or the second composition and/or the third composition may comprise one or more

surfactants. A surfactant can help to provide an emulsion. The first composition and/or the second composition and/or the third composition may be in the form of an emulsion.

The first composition and/or the second composition and/or the third composition may be in the form of a cream or gel. The first composition and/or the second composition and/or the third composition may have a lamellar structure and/or may have a gel network. The first composition and/or the second composition and/or the third composition may comprise micelles comprising a hydrophobic phase (see the description of the hydrophobic phase more below).

The first composition and/or the second composition and/or the third composition may comprise from 0.001% to 10%, or from 0.1% to 8%, or from 0.5% to 5%, or from 0.4% to 2%, or from 0.8% to 1.5% of the one or more surfactants by total weight of the respective first composition and/or the second composition and/or the third composition.

The first composition and/or the second composition and/or the third composition may comprise one or more surfactants which are selected from the group consisting of anionic surfactants, non-ionic surfactants, amphoteric surfactants, zwitterionic surfactants, cationic surfactants, and mixtures thereof. The one or more surfactants of the first composition and/or the second composition and/or the third composition can be useful for stabilising a hydrophobic phase in the first composition and/or the second composition, e.g. for stabilising the gel network and/or lamellar structure.

The first composition and/or the second composition and/or the third composition may comprise an anionic surfactant. The anionic surfactant of the first composition and/or the second composition and/or the third composition may be sodium lauryl sulfate or sodium laureth sulfate.

The one or more surfactants of the first composition and/or the second composition and/or the third composition may be a non-ionic surfactant. The non-ionic surfactant may be selected from the group consisting of lanolin alcohol, and polyoxyethylene ethers of fatty alcohols, and mixtures thereof. The non-ionic surfactant may be preferably ceteareth-n, wherein n is from 2 to 100, or from 10 to 30. When the one or more surfactants of the first composition and/or the second composition and/or the third composition are non-ionic, precipitation of others ingredients of the first composition and/or the second composition and/or the third composition can be prevented.

The first composition and/or the second composition and/or the third composition may comprise from 0.001 % to 5%, or from 0.01 % to 3%, or from 0.01 % to 1%, or from 0.05% to 1%, or from 0.1% to 0.5%, or from 0.1% to 0.3% of a non-ionic surfactant by total weight of the respective first composition and/or the second composition and/or the third composition. The non-ionic surfactant of the first composition and/or the second composition and/or the third composition may be selected from the group consisting of lanolin alcohol, and polyoxyethylene ethers of fatty alcohols, and mixtures thereof.

The non-ionic surfactant of the first composition and/or the second composition and/or the third composition may be a castor oil having polyethylene glycol ether groups or polypropylene glycol ether groups. The polyethylene glycol ether groups of the non-ionic surfactant may be ethers of PEG-n groups, wherein n is an integer of from 2 to 12, or from 2 to 10, or from 3 to 8. When the total M.Wt. of polyethylene glycol ether groups is below 400 Da, the mixing of the first composition and/or the second composition and/or the third composition can be eased.

The polypropylene glycol ether groups may be ethers of PPG-n groups, wherein n is an integer of from 2 to 60, or from 10 to 50, or from 20 to 40. The polyethylene glycol ether groups or polypropylene glycol ether groups may be selected from the group consisting of: PPG-4, PPG-6, PEG-5, PEG-6, PEG-8, and mixtures thereof. The first composition and/or the second composition and/or the third composition may comprise PEG-40 Hydrogenated Castor Oil and/or PEG-60 Castor Oil and/or PEG-35 Castor Oil as non-ionic surfactant.

## Thickening agent

The first composition and/or the second composition and/or the third composition may comprise one or more thickening agents. Thickening agents can help to provide the desired rheology for the first composition and/or the second composition and/or the third composition, which is useful in terms of mixing and anti-drip. The first composition and/or the second composition and/or the third composition may comprise from 0.01% to 5% of the one or more thickening agents by total weight of the respective first composition and/or the second composition and/or the third composition. The one or more thickening agents of the first composition and/or the second composition and/or the third composition may be a thickening polymer.

The first composition and/or the second composition and/or the third composition may comprise from 0.1% to 2% of a thickening polymer by total weight of the respective first composition and/or the second composition and/or the third composition. The thickening polymer of the first composition and/or the second composition and/or the third composition may be an associative polymer. The thickening polymer of the first composition and/or the second composition and/or the third composition may also serve as conditioning agents, as described hereinabove.

## Hydrophobic phase

The first composition and/or the second composition and/or the third composition may comprise a hydrophobic

phase. The hydrophobic phase of the first composition and/or the second composition and/or the third composition may be selected from the group consisting of fatty alcohols, fatty acids, and mixtures thereof. The fatty alcohols and/or fatty acids may comprise from 10 to 30, or from 12 to 20, or from 16 to 18 carbon atoms. The hydrophobic phase of the first composition and/or the second composition and/or the third composition may comprise two different fatty alcohols. The hydrophobic phase of the first composition and/or the second composition and/or the third composition may comprise two different fatty alcohols, both comprising from 10 to 14 carbons.

Pigment

The second and/or the third composition may comprise one or more pigments. The one or more pigments of the third composition may be a colored pigment which imparts color effects to the third composition or to the hair.

The one or more pigments of the second and/or the third composition may be a white pigment, such as, for example, titanium dioxide or zinc oxide. Alternatively, the one or more pigments of the second and/or the third composition may be a black pigment, such as, for example, iron oxide black. Alternatively, the one or more pigments of the second and/or the third composition may be a colored pigment, such as, for example, ultra-marine or iron oxide red, or a lustre pigment, or a metal effect pigment, or a pearlescent pigment, and/or a fluorescent or phosphorescent pigment.

The one or more pigments of the third composition may be colored or a non-white pigment. The one or more pigments of the third composition may be selected from the group consisting of metal oxides, hydroxides and oxide hydrates, mixed phase pigments, sulfur-containing silicates, metal sulfides, complex metal cyanides, metal sulfates, chromates and molybdates, the metals themselves (bronze pigments), and combinations thereof. The one or more pigments of the third composition may be selected from the group consisting of are titanium dioxide (CI 77891), black iron oxide (CI 77499), yellow iron oxide (CI 77492), red and brown iron oxide (CI 77491), manganese violet (CI 77742), ultramarine (sodium aluminium sulfosilicates, CI 77007, Pigment Blue 29), chromium oxide hydrate (CI 77289), Prussian blue (ferric ferrocyanide, CI 77510), carmine (cochineal), External Violet 2 (CI 69730), and mixtures thereof.

The one or more pigments of the second and/or the third composition may be selected from the group consisting of iron oxide, titanium dioxide, mica, borosilicate, and combinations thereof. The pigment of the second and/or the third composition may comprise an iron oxide ($Fe_2O_3$) pigment. The one or more pigments of the second and/or the third composition may comprise a combination of mica and titanium dioxide.

Preservative

The first composition and/or the second composition and/or the third composition may comprise at least one preservative and/or a mixture of preservatives. The first composition and/or the second composition and/or the third composition may comprise from 0.01% to 1% preservative, or from 0.1% to 0.5% preservative by total weight of the respective first composition and/or the second composition and/or the third composition. The preservative of the first composition and/or the second composition and/or the third composition may be selected from the group consisting of benzyl alcohol, phenoxyethanol, 1,3-bis(hydroxymethyl)-5,5-dimethylimidazolidine-2,4-dione, and mixtures thereof. The first composition and/or the second composition and/or the third composition may comprise at least one preservative; and wherein the preservative may be selected from the group consisting of benzyl alcohol, phenoxyethanol, and mixtures thereof; or wherein the preservative may be a mixture of benzyl alcohol and phenoxyethanol. The first composition and/or the second composition and/or the third composition may be substantially free of benzoate compounds. Indeed, having benzoate compounds can help to prevent instability and/or precipitation of the first composition and/or the second composition and/or the third composition. The first composition and/or the second composition and/or the third composition may be substantially free of parabens.

Perfume

The first composition and/or the second composition and/or the third composition may comprise a perfume. The first composition and/or the second composition and/or the third composition may comprise from 0.001% to 2% of a perfume by total weight of the respective first composition and/or the second composition and/or the third composition. Perfume can provide an enhanced user experience by making the composition smell pleasant and/or invoke emotions tailored to the visual effects on the fibers, such as relaxing or exciting smells.

Alternatively, the first composition and/or the second composition and/or the third composition may be substantially free of perfume and/or fragrance. Some consumers prefer perfume-free compositions.

The perfume of the first composition and/or the second composition and/or the third composition may be an animal fragrance or a plant fragrance. The animal fragrance may be selected from the group consisting of musk oil, civet, castoreum, ambergris, and mixtures thereof.

The plant fragrance may be selected from the group consisting of nutmeg extract, cardomon extract, ginger

extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

The perfume of the first composition and/or the second composition and/or the third composition may be selected from the group consisting of acetophenone, adoxal, aldehyde C-12, aldehyde C-14, aldehyde C-18, allyl caprylate, ambroxan, amyl acetate, dimethylindane derivatives, $\alpha$-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, $\alpha$-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-$\alpha$-naphthyl ketone, methylphenylcarbinyl acetate, mugetanol, $\gamma$-nonalactone, octanal, phenyl ethyl acetate, phenyl-acetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, $\gamma$-undecalactone, undecenal, vanillin, veloutone, verdox, and mixtures thereof.

<u>Viscosity</u>

The first, second and third compositions may be, independently from one another, prepared as so called thin liquids or creams. The first composition may be prepared as a spray.

Each of the first, second and third compositions may have a viscosity which induces a shear stress of from 20 to 200 Pa at 10 $s^{-1}$ as measured according to the viscosity test method.

The first or second or third composition may have a viscosity which induces a shear stress of from 60 to 200 Pa at 10 $s^{-1}$ as measured according to the viscosity test method.

The first or second or third composition may have a viscosity which induces a shear stress of from 20 to 180 Pa at 10 $s^{-1}$ as measured according to the viscosity test method.

Each of the first, second and third compositions may have a viscosity which induces a shear stress of from 20 to 60 Pa at 10 $s^{-1}$, when the first or second or third composition is applied to the hair with a container to which a nozzle or a separate applicator device such as a comb or a brush is attached.

The first or second or third composition may have a viscosity which induces a shear stress of from 30 to 200 Pa at 10 $s^{-1}$, more preferably from 100 to 200 Pa at 10 $s^{-1}$, even more preferably from 130 to 180 Pa at 10 $s^{-1}$ when the first or second or third composition is applied to the hair with a brush and bowl applicator or with the hands or fingers of the user.

Whilst not being bound by theory, it is believed that the provision of the first or second or third compositions having viscosity values as described hereinabove enables the first or second or third composition enables the first or second or third composition to be easily applied and distributed along the entire remaining hair length with minimal to no dripping from the hair.

<u>Application means</u>

Each of the first, second and third compositions may be applied to the hair with a brush and bowl applicator. Alternatively, the first or second and/or third composition may be applied to the hair with a brush and bowl applicator. Alternatively, the first or second and/or third composition may be applied to the hair with the hands and fingers of the user. Alternatively, the first or second and/or third composition may be applied to the hair with a container to which a nozzle or a separate applicator device such as a comb is attached.

The application means may also include means which assist in achieving particular effects such as highlighting such as highlighting combs, brushes and tools, foils and highlighting caps. Additional application means technology can be used to assist in the penetration of each of the first, second and third compositions into the hair. Examples of such technology include heating devices, ultraviolet light devices and ultrasound devices.

Kit

A second aspect of the present invention is related to a kit for treating un-pigmented hair. A kit for treating un-pigmented hair, preferably gray or white hair, comprises a first, a second and a third compositions packaged in different containers or in a same container in different compartments. The first composition comprises one or more heavy metal ion sequestrants as defined hereinabove. The second composition comprises one or more weak acids having a pKa from 3 to 5 as defined hereinabove. The third composition comprises one or more direct dyes as defined hereinabove.
The kit may further comprise a shampoo and a conditioner packaged in different containers.
The kit may further comprise an applicator. The applicator may be a brush and bowl applicator. Alternatively, the applicator may be a nozzle which may be attached to one of the containers comprised in the kit or a separate applicator device such as a comb or a brush. Such combs and brushes can be adapted in order to achieve particular effects, whether it is quick and even coverage or root/hairline touch up, or highlights or streaks.
Alternatively, one of the containers may be provided with a comb attached to or instead of the dispensing nozzle whereby the product is dispensed through hollow tines and dispensing apertures located in the comb tines. The comb tines may be provided with single or multiple openings along the tines to improve product application and evenness especially root to tip. Product dispensation can be achieved by mechanical pressure applied to the container for example delaminating bottles or any of the mechanisms described hereinabove.
The comb may be provided on the container such as to facilitate easy application and may be positioned vertically (so called verticomb) or at an angle to allow the consumer to access all areas.

EXAMPLES

The following examples are non-limiting examples of the first, second and third compositions, the method and kit of the present invention. The examples are given solely for the purpose of illustration, and are not to be construed as limitations of the present invention since many variations thereof are possible without departing from the spirit and scope of the invention, which would be recognized by one of ordinary skill in the art. All concentrations are listed as weight percent (% wt.), unless otherwise specified. "QSP" means sufficient quantity for 100% or for 100 g.

First Composition Examples 1 to 7

The following first compositions are prepared.

| **Ingredients** | **Ex.1** (% wt.) | **Ex.2** (% wt.) | **Ex.3** (% wt.) | **Ex.4** (% wt.) | **Ex.5** (% wt.) | **Ex.6** (% wt.) | **Ex.7** (% wt.) |
|---|---|---|---|---|---|---|---|
| Heavy metal ion sequestrant | EDDG | HPDDS | EDDS | GADS | EDDHA | HBED | EDTMP |
| | 1.5 | 2.0 | 1.6 | 2.5 | 0.5 | 5 | 10 |
| Amodimethicone and Cetrimonium chloride | 2.0 | 2.0 | 1.9 | 1.7 | 1.8 | 2.0 | 2.0 |
| Polyquaternium-11 | 1.3 | 1.3 | 1.3 | 1.3 | 1.3 | 1.3 | 1.3 |
| Phenoxyethanol and ethylhexylglycerin | 1.0 | 1.0 | 1.0 | 1.0 | 1.0 | 1.0 | 1.0 |
| Benzyl alcohol | 0.4 | 0.5 | 0.4 | 0.5 | 0.4 | 0.4 | 0.4 |
| Panthenol DL 56% solution | 0.2 | 0.2 | 0.2 | 0.2 | 0.2 | 0.2 | 0.2 |
| Panthenyl ethyl ether | 0.1 | 0.1 | 0.1 | 0.1 | 0.1 | 0.1 | 0.1 |
| Water purified | QSP | QSP | QSP | QSP | QSP | QSP | QSP |
| EDDG: ethylenediamine-N,N'-diglutaric acid; HPDDS: 2-hydroxypropylenediamine-N,N'-disuccinic acid; EDDS: ethylenediamine-N,N'-disuccinic acid; GADS: glycinamide-N,N'-disuccinic acid; EDDHA: ethylenediamine-N,N'-bis(ortho-hydroxyphenyl acetic acid); HBED: N,N'-bis(2-hydroxybenzyl)ethylenediamine-N,N'-diacetic acid; EDTMP: ethylene-diaminotetra-(methylenephosphonic acid) | | | | | | | |

Second Composition Examples 8 to 14

The following second compositions are prepared.

| **Ingredients** | **Ex.8** (% wt.) | **Ex.9** (% wt.) | **Ex.10** (% wt.) | **Ex.11** (% wt.) | **Ex.12** (% wt.) | **Ex.13** (% wt.) | **Ex.14** (% wt.) |
|---|---|---|---|---|---|---|---|
| Weak acid | Maleic acid | Succinic acid | L-tartric acid | Acotinic acid | Malonic acid | Citric acid | Isocitric acid |
| | 5.0 | 2.0 | 10.0 | 12.0 | 5.0 | 2.5 | 3.0 |
| Propylene glycol | 8.0 | 8.0 | 8.0 | 8.0 | 8.0 | 8.0 | 8.0 |
| Ethoxydiglycol | 3.0 | 3.0 | 3.0 | 3.0 | 3.0 | 3.0 | 3.0 |
| Cetearyl alcohol and dicetylphosphate and ceteth-10 phosphate | 7.0 | 7.0 | 7.0 | 7.0 | 7.0 | 7.0 | 7.0 |
| Ceteraryl alcohol | 0.4 | 0.4 | 0.4 | 0.4 | 0.4 | 0.4 | 0.4 |
| Sodium hydroxide | 0.3 | 0.3 | 0.3 | 0.3 | 0.3 | 0.3 | 0.3 |
| Xanthan gum | 0.1 | 0.1 | 0.1 | 0.1 | 0.1 | 0.1 | 0.1 |
| 2-phenoxyethanol | 0.5 | 0.5 | 0.5 | 0.5 | 0.5 | 0.5 | 0.5 |
| Ascorbic acid | 0.3 | 0.3 | 0.3 | 0.3 | 0.3 | 0.3 | 0.3 |
| Sodium sulfite | 0.4 | 0.4 | 0.4 | 0.4 | 0.4 | 0.4 | 0.4 |
| Disodium EDTA | 0.1 | 0.1 | 0.1 | 0.1 | 0.1 | 0.1 | 0.1 |
| Water purified | QSP | QSP | QSP | QSP | QSP | QSP | QSP |

Third Composition Examples 15 to 21

The following third compositions are prepared.

| **Ingredients** | **Ex.15** (% wt.) | **Ex.16** (% wt.) | **Ex.17** (% wt.) | **Ex.18** (% wt.) | **Ex.19** (% wt.) | **Ex.20** (% wt.) | **Ex.21** (% wt.) |
|---|---|---|---|---|---|---|---|
| HC Red No. 3 CAS 2871-01-4 | 0 | 0 | 0 | 0 | 0.009 | 0.01 | 0.01 |
| HC Blue No. 16 CAS 502453-61-4 | 0 | 0.01 | 0 | 0 | 0.005 | 0.01 | 0.01 |
| HC Blue No. 2 CAS 33229-34-4 | 0.02 | 0.01 | 0.02 | 0.01 | 0.007 | 0.01 | 0.01 |
| External Violet 2 (CI 60730) CAS 4430-18-6 | 0 | 0.01 | 0.01 | 0.03 | 0.03 | 0.02 | 0.03 |
| Disperse Violet 1 CAS 128-95-0 | 0.01 | 0.01 | 0.01 | 0.01 | 0 | 0.001 | 0 |
| Basic Red 76 CAS 68391-30 | 0 | 0 | 0 | 0.01 | 0.01 | 0.01 | 0 |
| Propylene glycol | 8.0 | 8.0 | 8.0 | 8.0 | 8.0 | 8.0 | 8.0 |
| Ethoxydiglycol | 3.0 | 3.0 | 3.0 | 3.0 | 3.0 | 3.0 | 3.0 |
| Cetearyl alcohol and dicetylphosphate and ceteth-10 phosphate | 7.0 | 7.0 | 7.0 | 7.0 | 7.0 | 7.0 | 7.0 |
| Ceteraryl alcohol | 0.4 | 0.4 | 0.4 | 0.4 | 0.4 | 0.4 | 0.4 |
| Sodium hydroxide | 0.3 | 0.3 | 0.3 | 0.3 | 0.3 | 0.3 | 0.3 |
| Xanthan gum | 0.1 | 0.1 | 0.1 | 0.1 | 0.1 | 0.1 | 0.1 |
| 2-phenoxyethanol | 0.5 | 0.5 | 0.5 | 0.5 | 0.5 | 0.5 | 0.5 |
| Ascorbic acid | 0.3 | 0.3 | 0.3 | 0.3 | 0.3 | 0.3 | 0.3 |
| Sodium sulfite | 0.4 | 0.4 | 0.4 | 0.4 | 0.4 | 0.4 | 0.4 |

(continued)

| Ingredients | Ex.15 (% wt.) | Ex.16 (% wt.) | Ex.17 (% wt.) | Ex.18 (% wt.) | Ex.19 (% wt.) | Ex.20 (% wt.) | Ex.21 (% wt.) |
|---|---|---|---|---|---|---|---|
| Disodium EDTA | 0.1 | 0.1 | 0.1 | 0.1 | 0.1 | 0.1 | 0.1 |
| Water purified | QSP | QSP | QSP | QSP | QSP | QSP | QSP |

**Method Examples M1 to M5**

Material needed

**Hair strands**

Virgin hair strands having a width of 1 cm and a length of 20 cm.

The virgin hair strands have varying level of un-pigmented hair from dark brown hair to light blond hair comprising from 10% to 100% wt. of un-pigmented hair, preferably gray or white hair.

Available from *International Hair Importers* & *Products*, Glendale, NY
Mass: 4.0 g ± 0.1g
Characteristics: cysteic acid: 17.4 - 18.1 μmol/g hair; medullated hair, ϕ: 60-80 μm

The following method examples were prepared:

**Method Example M1 (hair strands only treated with the first composition)**

The virgin hair strands were wet until damp. The first composition of Example 3 was prepared. An amount of 2.0 g of the first composition of Example 3 was sprayed all over the wet virgin hair strands. The hair strands were blow dried with a conventional blow dryer, e.g. Vidal Sassoon Ceramic blow drier 1875W. The first composition of Example 3 was left on the hair for 15 minutes. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

**Method Example M2 (hair strands only treated with the second composition)**

The second composition of Example 13 was prepared. 4.0 g of the second composition of Example 13 per g of hair were applied to the virgin hair strands. The virgin hair strands were completely covered with the second composition of Example 13. The second composition of Example 13 was left on the hair for 15 minutes at 30°C. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

**Method Example M3A (hair strands only treated with the third composition of Example 15 - Violet shade)**

The third composition of Example 15 was prepared. 4.0 g of the third composition of Example 15 per g of hair were applied to the virgin hair strands. The third composition of Example 15 was left on the hair for 15 minutes at 30°C. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

**Method Example M3B (hair strands only treated with the third composition of Example 19 - Blue shade)**

The third composition of Example 19 was prepared. 4.0 g of the third composition of Example 19 per g of hair were applied to the virgin hair strands. The third composition of Example 19 was left on the hair for 15 minutes at 30°C. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

**Method Example M13A (Treatment M1 + M3A)**

The virgin hair strands were wet until damp. The first composition of Example 3 was prepared. An amount of 2.0 g of the first composition of Example 3 was sprayed all over the wet virgin hair strands. The hair strands were blow dried with a conventional blow dryer, e.g. Vidal Sassoon Ceramic blow drier 1875W. The first composition of Example 3 was left on the hair for 15 minutes. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

The third composition of Example 15 was prepared. 4.0 g of the third composition of Example 15 per g of hair were applied to the hair strands treated with the first composition of Example 3. The third composition of Example 15 was left on the hair for 15 minutes at 30°C. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

**Method Example M23A (Treatment M2 + M3A)**

The second composition of Example 13 was prepared. 4.0 g of the second composition of Example 13 per g of hair were applied to the virgin hair strands. The virgin hair strands were completely covered with the second composition of Example 13. The second composition of Example 13 was left on the hair for 15 minutes at 30°C. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

The third composition of Example 15 was prepared. 4.0 g of the third composition of Example 15 per g of hair were applied to the hair strands treated with the second composition of Example 13. The third composition of Example 15 was left on the hair for 15 minutes at 30°C. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

**Method Example M4 according to the invention**

The virgin hair strands were wet until damp. The first composition of Example 3 was prepared. An amount of 2.0 g of the first composition of Example 3 was sprayed all over the wet virgin hair strands. The hair strands were blow dried with a conventional blow dryer, e.g. Vidal Sassoon Ceramic blow drier 1875W. The first composition of Example 3 was left on the hair for 15 minutes. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair.

The conditioner was applied and milked from hair for 30 seconds, let sit for 30 seconds, and the hair strands were rinsed for 1 minute. The hair strands were blow dried with a conventional blow dryer 1875W.

The second composition of Example 13 was prepared. 4.0 g of the second composition of Example 13 per g of hair were applied to the hair strands treated with the first composition of Example 3. The hair strands were completely covered with the second composition of Example 13. The second composition of Example 13 was left on the hair for 15 minutes at 30°C. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

The third composition of Example 15 was prepared. 4.0 g of the third composition of Example 15 per g of hair were applied to the hair strands treated with the first composition of Example 3 and then the second composition of Example 13. The third composition of Example 15 was left on the hair for 15 minutes at 30°C. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

**Method Example M13B (Treatment M1 + M3B)**

The virgin hair strands were wet until damp. The first composition of Example 3 was prepared. An amount of 2.0 g of the first composition of Example 3 was sprayed all over the wet virgin hair strands. The hair strands were blow dried with a conventional blow dryer, e.g. Vidal Sassoon Ceramic blow drier 1875W. The first composition of Example 3 was left on the hair for 15 minutes. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

The third composition of Example 19 was prepared. 4.0 g of the third composition of Example 19 per g of hair were applied to the hair strands treated with the first composition of Example 3. The third composition of Example 19 was left on the hair for 15 minutes at 30°C. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

**Method Example M23B (Treatment M2 + M3B)**

The second composition of Example 13 was prepared. 4.0 g of the second composition of Example 13 per g of hair were applied to the virgin hair strands. The virgin hair strands were completely covered with the second composition of Example 13. The second composition of Example 13 was left on the hair for 15 minutes at 30°C. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

The third composition of Example 19 was prepared. 4.0 g of the third composition of Example 19 per g of hair were applied to the hair strands treated with the second composition of Example 13. The third composition of Example 19 was left on the hair for 15 minutes at 30°C. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

**Method Example M5 according to the invention**

The virgin hair strands were wet until damp. The first composition of Example 3 was prepared. An amount of 2.0 g of the first composition of Example 3 was sprayed all over the wet virgin hair strands. The hair strands were blow dried with a conventional blow dryer, e.g. Vidal Sassoon Ceramic blow drier 1875W. The first composition of Example 3 was left on the hair for 15 minutes. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

The second composition of Example 13 was prepared. 4.0 g of the second composition of Example 13 per g of hair were applied to the hair strands treated within the first composition of Example 3. The hair strands were completely covered with the second composition of Example 13. The second composition of Example 13 was left on the hair for 15 minutes at 30°C. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

The third composition of Example 19 was prepared. 4.0 g of the third composition of Example 19 per g of hair were applied to the hair strands treated with the first composition of Example 3 and then with the second composition of Example 13. The third composition of Example 19 was left on the hair for 15 minutes at 30°C. The hair strands were subsequently treated with Wella Luxe Oil Keratin Protect shampoo. An amount of 0.1 g of the shampoo per g of hair was applied to the hair. The shampoo was lathered on hair for 30 seconds, and the hair strands were rinsed for 1 minute. The hair strands were subsequently treated with Wella Luxe Oil Keratin Restore Mask conditioner. An amount of 0.1 g of the conditioner per g of hair was applied to the hair. The conditioner was applied and milked from hair for 30 seconds, let sit for 30 seconds, and the hair strands were rinsed for 1 minute. The hair strands were blow dried with a conventional blow dryer 1875W.

EXPERIMENTAL

<u>Determining the amount of un-pigmented hair</u>

An experienced colorist or even a home consumer can use their visual perception to study respectively a client's hair or his own hair and determine the percentage of pigmented versus unpigmented hair at 25%, 50%, 75%, 80%, 90%, 95% & 100% blends of un-pigmented (gray or white) hair.

Typically, the colorist or the home consumer may compare the hair to known hair samples or depictions.

For example, the "blend finder" or top center panel in the ARTec Systems Group, Inc. enamels® Consultation Center, provides a scale to measure against. The neutral series represents natural pigmented hair.

Assessing the level of un-pigmented hair, or gray hair may be useful when the colorist or the home consumer selects a third composition to deliver desired properties resulting across both the white and pigmented hair of their client.

<u>Viscosity test method</u>

The viscosity of a composition is measured using a TA Instruments AR 2000 Rheometer or equivalent device equipped with a Peltier plate and a 6 cm flat acrylic plate with cross hatchings. The instrument is calibrated according to the manufacturer's instructions and the Peltier plate is set at 25.0°C. The cone is raised to a position approximately 4.5 cm above the plate. Immediately after the mixing, approximately 10.0 g of the mixture is transferred gently onto the centre of the Peltier plate using a spatula. The cone is lowered to obtain the specified gap between the tip of the cone and the upper surface of the Peltier plate. The gap setting is specified by the manufacturer of the cone and is typically approximately 1000 microns. The rheometer is programmed to operate in rotational mode with the shear stress ramped from 0.1 to 600 Pa over a period of 4 minutes, termination at 1000 reciprocal seconds. Rotation is initiated immediately after the specified gap is established. Viscosity data collected during the measurement period are shear stress (Pa) plotted as a function of shear rate ($s^{-1}$).

<u>Scanning Electron Microscopy and Energy dispersive *X*-ray spectroscopy</u>

About 50 hair fibers from an untreated human hair strand have been observed through a Scanning Electron Microscope (Hitachi S-300N). Any presence of white deposits has been determined. The SEM is a type of electron

microscope that produces images of a sample by scanning it with a focused beam of electrons.

Significant white deposits 11 have been seen on an untreated human hair fiber 10 before any treatment according to the method of the present invention, i.e. before any application of each of the first, second and third compositions (see Fig. 1A).

About 50 human hair fibers that have been treated according to the method of Example M4 have been observed through a Scanning Electron Microscope (SEM). Presence of white deposits 11 has been determined.

Relatively lower levels of white deposits 11 in treated human hair fibers 20 have been observed compared to the levels of white deposits 11 for untreated human hair fibers 10 (see Figs. 1A-1B).

The white deposits have been analyzed by *Energy dispersive X*-ray spectroscopy (EDX, Brucker XFlash 6/60). Two main deposits have been identified: magnesium/silicone, and magnesium/aluminum.

Optical Microcopy

Between 10 and 20 un-pigmented human hair fibers have been observed with an optical microscope (Olympus BX-61, using a 40X magnification lens). Un-pigmented human hair fibers were selected in each human hair strand since the surface deposits in pigmented hair fibers were difficult to observe.

Surface deposits 22 have been observed on untreated human un-pigmented fibers 10 (see Fig. 2A).

Relatively lower levels of surface deposits 22 have been observed on un-pigmented human hair fibers 20 treated according to the method of Example **M4** compared to untreated un-pigmented human hair fibers (see Fig. 2B).

Inductively coupled plasma atomic spectroscopy

Levels of copper metal have been quantified in untreated and treated human hair strands using the inductively coupled plasma atomic spectroscopy.

Samples of 100 mg of untreated human hair fibers, and samples of 100 mg of human hair fibers treated respectively with Wella Luxe Oil Keratin Protect Shampoo, or according the method Example **M1** or **M2** were digested overnight with 2 mL of high purity concentrated nitric acid. The digestive mixture also contained 150 μL of 100 μg/g of Yttrium internal standard (Inorganic Ventures, Christianburg, VA, U.S.A.). Following digestion, samples were heated to 70-80°C for 1 hour, cooled to room temperature and diluted to 15 mL with deionized water. Each hair strand was analyzed in triplicate.

Metal content of each hair strand was determined by inductively coupled plasma atomic spectroscopy (ICP-OES) with an Optima 5300DV Optical Emission Spectrometer (PerkinElmer Life and Analytical Sciences, Shelton, CT, U.S.A.).

| Sample | Copper content (μg / g) |
|---|---|
| Untreated human hair strand | 17.8 |
| Human hair strand treated with Wella Luxe Oil Keratin Protect Shampoo | 16.4 |
| Human hair strand treated with the first composition of Example **3** | 14.4 |
| Human hair strand treated with the second composition of Example **13** | 15.8 |

When the human hair fibers are treated with the first composition comprising a heavy metal ion sequestrant, the level of copper remaining on the human hair fibers has been relatively reduced compared to untreated human hair fibers.

Goniophotometric Measurement - Single Fiber Luster

Untreated and treated human un-pigmented hair strands were analyzed through a goniophotometer in order to measure the luster/shine of the hair fibers.

The use of a goniophotometer in measuring the luster of single fibers impinges He-Ne laser beam (reference for this method can be found in K. Keis, K. R. Ramaprasad and Y. K. kamath, Studies of light scattering from ethnic hair fibers, J. Cosmet. Sci., 55, 49-63 (2004)). The reflected light is mapped using a software controlled photomultiplier detector. The reflection profile is then analyzed for its specular and diffuse components and luster is calculated using the formula:

$$\text{Luster, L(\%)} = [S/(S+D)]*[W^{std}_{1/2} / W^{sam}_{1/2}]\%100$$

Wherein:

S is the amount of specular light in the reflection profile;
D is the diffuse component,
$W_{1/2}$ values are the peak widths at half maximum for a black standard (*std*) and the sample (*sam*). Generally, 50 fibers are thus individually analyzed for better statistical control over the data.

Fifty replicates were run per untreated human un-pigmented hair strand, and per human unpigmented hair strand treated according to the method Example **M4.**

| Sample | Luster, L(%) |
|---|---|
| Untreated human un-pigmented hair strand | 6.93 |
| Human un-pigmented hair strand treated according to the method of Example **M4** | 7.84 |

Human un-pigmented hair fibers treated according to the method of Example **M4** versus untreated human un-pigmented hair fibers showed a significant increase of luster, and thus of the shine. The removal of surface deposits due to the application of the first and second compositions prior to the third composition has been translated into a relative increase of the shine.

Reflectance

*Procedure of Spectral Data Measurement*

M0 corresponds to the reference which is untreated hair strands: virgin hair strands
For M0 and each Method Example M1-M5, the reflectance of the hair fibers was measured on a Minolta CM3700A spectrophotometer with the following settings:

o Spectrophotometer Settings:

- Measurement Type - Reflectance
- Geometry di:8, de:8
- Specular Component - SCE (excluded)
- Measurement Area - MAV (8mm)
- UV Setting - 100% Full

The Base line color was established prior to any treatment to the hair. For each hair strand, the measurement was carried out by the spectrophotometer with minimum of 10 measurements taken along the length of the hair and on both sides.
Color measurements were first taken with the virgin hair strands (M0) and then taken after each individual step/treatment (as described above: M1, M2, M3A or M3B) and after the combination of each of the treatments (as described above and described in the legend below: M13A or M13B; M23A or M23B; M4 or M5)
Fig. 3 shows the color measurements made for:

- M0 - Reference: untreated virgin hair fibers
- M1: hair strands only treated with the first composition (Example 3)
- M2: hair strands only treated with the second composition (Example 13)
- M3A: hair strands only treated with a third composition (Example 15)
- M13A: hair strands only treated with the first and third compositions (Examples 3+15)
- M23A: hair strands only treated with the second and third compositions (Examples 13+15)
- M4: hair strands only treated with the first, second and third compositions (Examples 3+13+15)

Fig. 4 shows the color measurements made for:

- M0 - Reference: untreated virgin hair fibers
- M1: hair strands only treated with the first composition (Example 3)
- M2: hair strands only treated with the second composition (Example 13)
- M3B: hair strands only treated with a third composition (Example 19)
- M13B: hair strands only treated with the first and third compositions (Examples 3+19)
- M23B: hair strands only treated with the second and third compositions (Examples 13+19)
- M5: hair strands only treated with the first, second and third compositions (Examples 3+13+19)

*Spectral Data analysis*

The Color data was captured via the Minolta CM3700A spectrophotometer and using a SpectraMagic NX software.

The Reflectance was gathered at each individual wavelengths from 360 nm to 740 nm at a 10 nm interval.

The Overall color was analyzed via CIE L* a* b* generated at D65 light source. Analysis of color was based on individual color components in the CIE L* a* b* color space, wherein:

L* = lightness (L* = 0 yields black and L* = 100 indicates diffuse white)
a* = red/green (a*, negative values indicate green while positive values indicate red)
b* = yellow to blue (b*, negative values indicate blue and positive values indicate yellow)

Data for each individual measurement, and individual data points were then analyzed in the JMP statistics software package evaluating degree of change from the untreated hair color data. Measurements were evaluated in JMP as an Oneway Analysis of the color components using means comparisons for each pair, Student's T-test, i.e. a standard statistical analysis method (alpha 0.05), and evaluating for significantly different results though a connecting letters report. Color reflectance was evaluated by plotting the means of color reflectance data against wavelength from the data generated thought the SpecraMagic NX software (via the Minolta Spectrophotometer) on the treatments outlined on the matrix above.

*Interpretation*

Figs. 3 and 4 show the color reflectance for single and/or combination of treatment steps. Fig. 3 shows the color reflectance of hair strands only treated respectively with the first composition (M1), the second composition (M2) and the third composition (M3A).

As compared with the reference M0, the treatment of virgin hair strands with either the first composition of Example 3 or the second composition of Example 13 has only a slight effect on the reflectance of the hair fibers.

It can only be observed a significant decrease of the reflectance of the hair fibers when applying the third composition of Example 15 (M3A).

When combining a treatment involving the first and the third composition (M13A) or a treatment involving the second and the third composition (M23A), no further decrease of the reflectance of the hair fibers is obtained compared to a treatment with only the third composition of Example 15 (M3A).

However, it has been surprisingly discovered that when the virgin hair fibers have been treated with the first, second and third compositions successively (M4) that the reflectance is further decreased compared to the treatment M3A only. In that case, a significant decrease of the reflectance is observed from 530 nm to 630 nm. The color on the treated hair strands appear less yellow-orange compared to untreated virgin hair strands since there are less reflections at these wavelengths versus untreated virgin hair strands.

Fig. 4 shows the color reflectance of hair strands only treated respectively with the first composition (M1), the second composition (M2) and the third composition (M3B).
As compared with the reference M0, the treatment of virgin hair strands with either the first composition of Example 3 or the second composition of Example 13 has only a slight effect on the reflectance of the hair fibers.

It can only be observed a significant decrease of the reflectance of the hair fibers when applying the third composition of Example 19 (M3B).

A treatment combining the first and the third composition (M13B) or combining the second and the third composition (M23B) does not lead to a further decrease of the reflectance of the hair fibers compared to the treatment with the third composition of Example 19 only (M3B).

However, it has been surprisingly discovered that when the virgin hair fibers have been treated with the first, second and third compositions successively (M5) that the reflectance is further decreased compared to the treatment with the third composition of Example 19 only (M3B). In that case, a significant decrease of the reflectance is observed from 530 nm to 670 nm. The color on the treated hair strands appear less yellow-orange-red compared to untreated

virgin hair strands since there are less reflections at these wavelengths versus untreated virgin hair strands.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method for treating and coloring hair, preferably human hair, comprising un-pigmented hair, preferably gray or white hair, comprising:

   (a) Providing a first composition comprising one or more heavy metal ion sequestrants;
   (b) Applying the first composition to the hair;
   (c) Providing a second composition comprising one or more weak acids having a pKa from 2.5 to 5, preferably from 2.5 to 4.5, more preferably from 2.5 to 3.5;
   (d) Applying the second composition to the hair;
   (e) Providing a third composition wherein the third composition comprises one or more direct dyes which are selected from the group consisting of a nitro dye, a disperse dye, a cationic dye, an acid dye, a basic dye, a neutral azo dye and mixtures thereof, wherein the third composition is able to decrease the reflectance of un-pigmented hair in a wavelength range from 530 nm to 670 nm; and
   (f) Applying the third composition to the hair.

2. The method according to claim 1, wherein the method is able to decrease the reflectance of unpigmented hair in a wavelength range from 530 nm to 670 nm, preferably from 540 nm to 670 nm, more preferably from 560 nm to 650 nm, even more preferably from 570 nm to 630 nm, further than when only applying the third composition to hair.

3. The method according to any preceding claims, wherein the one or more heavy metal ion sequestrants are selected from the group consisting of nitrilotriacetic acid, ethylenediaminotetracetic acid, ethylenetriamine pentaacetic acid, ethylenediamine-N,N'-disuccinic acid (EDDS), ethylenediamine-N,N'-diglutaric acid (EDDG), 2-hydroxypropylenediamine-N,N'-disuccinic acid (HPDDS), glycinamide-N,N'-disuccinic acid (GADS), ethylenediamine-N,N'-bis(ortho-hydroxyphenyl acetic acid) (EDDHA), N,N'-bis(2-hydroxybenzyl)ethylenediamine-N,N'-diacetic acid (HBED), aminotri-(methylenephosphonic acid), ethylene-diaminotetra-(methylenephosphonic acid) (EDTMP), diethylene-triamine-penta-(methylenephosphonic acid) (DTPMP), salts thereof, and mixtures thereof.

4. The method according to claim 3, wherein the one or more heavy metal ion sequestrants are selected from the group consisting of ethylenediamine-N,N'-disuccinic acid (EDDS), salts thereof, and mixtures thereof.

5. The method according to any preceding claims, wherein the total amount of the one or more heavy metal ion sequestrants is from 0.005% to 20%, preferably from 0.01% to 10%, more preferably from 0.05% to 2% by total weight of the first composition.

6. The method according to any preceding claims, wherein the one or more weak acids of the second composition are able to bind with a fatty acid salt.

7. The method according to any preceding claims, wherein the one or more weak acids of the second composition are selected from the group consisting of succinic acid, maleic acid, malonic acid, glycolic acid, acotinic acid, 2-picolinic acid, L-tartric acid, D-tartric acid, citric acid, isocitric acid, ascorbic acid, lactic acid, benzoic acid, oxalic acid, acetic acid, formic acid, propionic acid and mixtures thereof.

8. The method according to claim 7 wherein the one or more weak acids of the second composition are selected from the group consisting of succinic acid, maleic acid, malonic acid, glycolic acid, acotinic acid, 2-picolinic acid, L-tartric acid, citric acid, isocitric acid, and mixtures thereof.

9. The method according to any preceding claims, wherein the total amount ot the one or more weak acids is from 0.5% to 20%, preferably from 0.1% to 10%, more preferably from 1% to 5% by total weight of the second composition.

10. The method according to any preceding claims, wherein the third composition comprises one or more direct dyes

which are selected from the group consisting of nitro dye, cationic dye, acid dye, basic dye and mixtures thereof; and wherein the third composition is applied to the hair comprising from 25% to 90%, preferably from 50% to 85%, more preferably from 75% to 85% of un-pigmented hair.

**11.** The method according to any of the claims 1-9, wherein the third composition comprises one or more direct dyes which are selected from the group consisting of nitro dye, disperse dye and mixtures thereof; and wherein the third coloring composition is applied to hair comprising from 80% to 100%, preferably from 90% to 100%, more preferably from 95% to 100% of un-pigmented hair.

**12.** The method according to any preceding claims, wherein the total amount of the one or more direct dyes is from 0.001% to 5.0%, preferably from 0.003% to 2.5%, more preferably from 0.005% to 1.5%, even more preferably from 0.005% to 1.0% by total weight of the third composition.

**13.** The method according to any preceding claims, wherein one or more of the steps chosen from the steps (b), (d) and (f) is followed by treating the hair with a treatment selected from the group consisting of rinsing, shampooing, conditioning and combinations thereof.

**14.** A kit for treating un-pigmented hair, preferably gray or white hair, comprising a first, a second and a third compositions packaged in different containers or in a same container in different compartments;
wherein the first composition comprises one or more heavy metal ion sequestrants as defined in any of the preceding claims;
wherein the second composition comprises one or more weak acids having a pKa from 3 to 5 as defined in any of the preceding claims; and
wherein the third composition comprises one or more direct dyes as defined in any of the preceding claims.

**15.** The kit according to claim 14 further comprising a shampoo and a conditioner packaged in different containers.

**16.** Use of a first, a second and a third compositions as defined in any of the preceding claims for improving the luster and the light reflectance of un-pigmented hair.

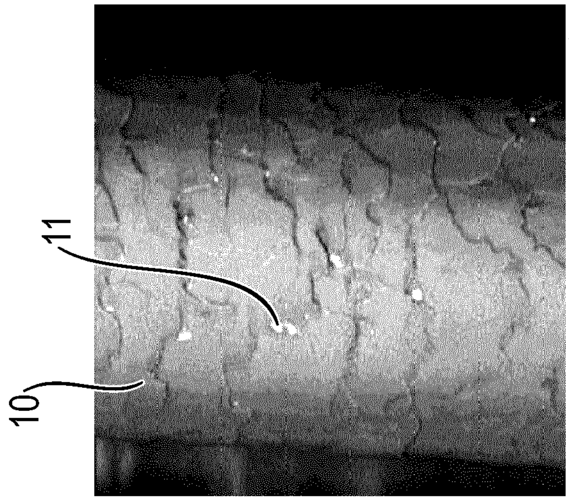


Fig. 1A

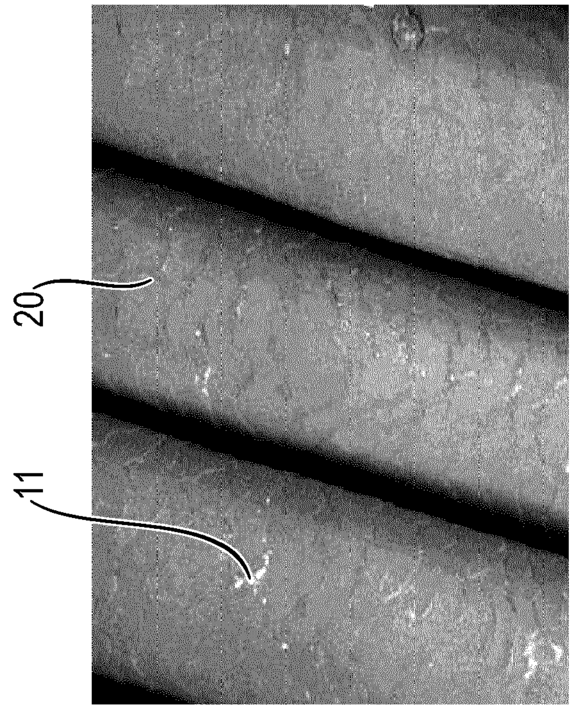


Fig. 1B

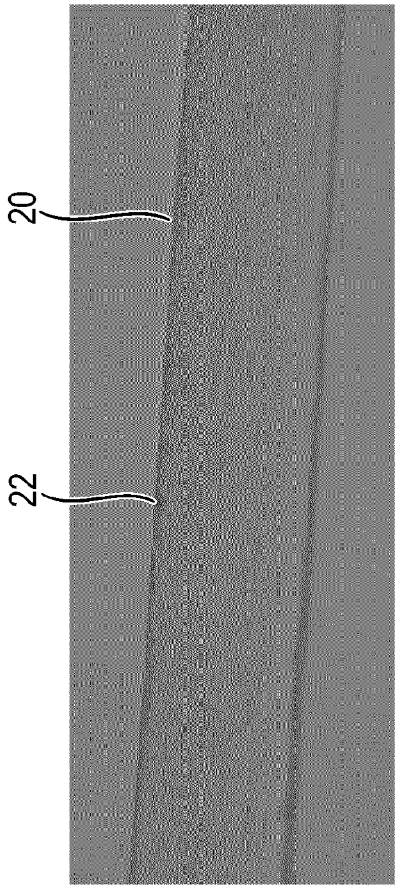


Fig. 2B

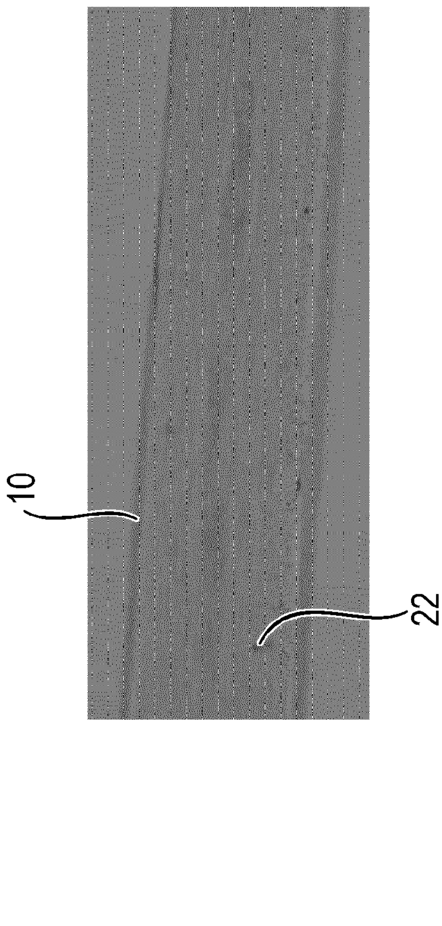


Fig. 2A

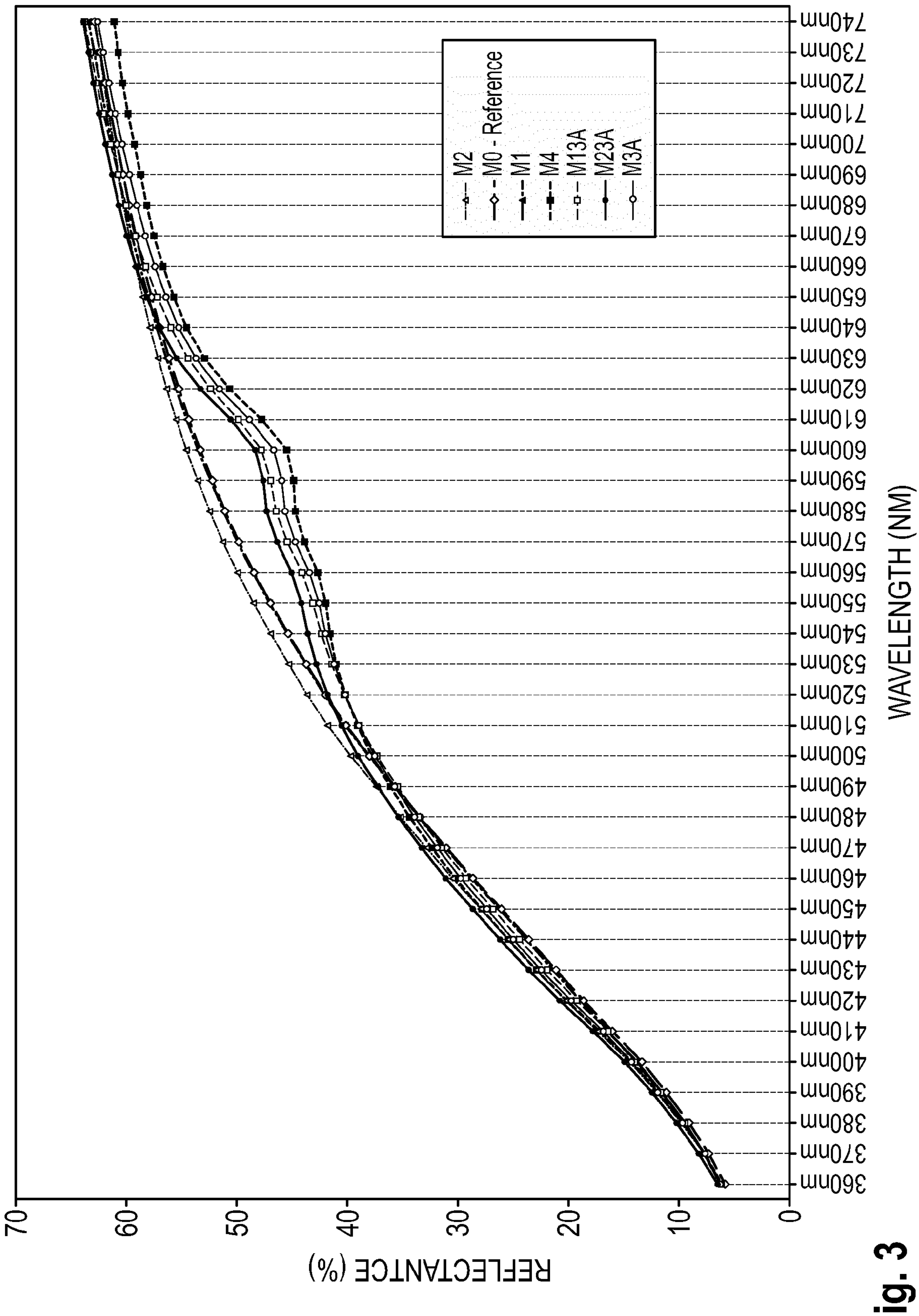
REFLECTANTCE (%)
0
10
20
30
40
50
60
70
WAVELENGTH (NM)
360nm
370nm
380nm
390nm
400nm
410nm
420nm
430nm
440nm
450nm
460nm
470nm
480nm
490nm
500nm
510nm
520nm
530nm
540nm
550nm
560nm
570nm
580nm
590nm
600nm
610nm
620nm
630nm
640nm
650nm
660nm
670nm
680nm
690nm
700nm
710nm
720nm
730nm
740nm
M2
M0 - Reference
M1
M4
M13A
M23A
M3A

Fig. 3

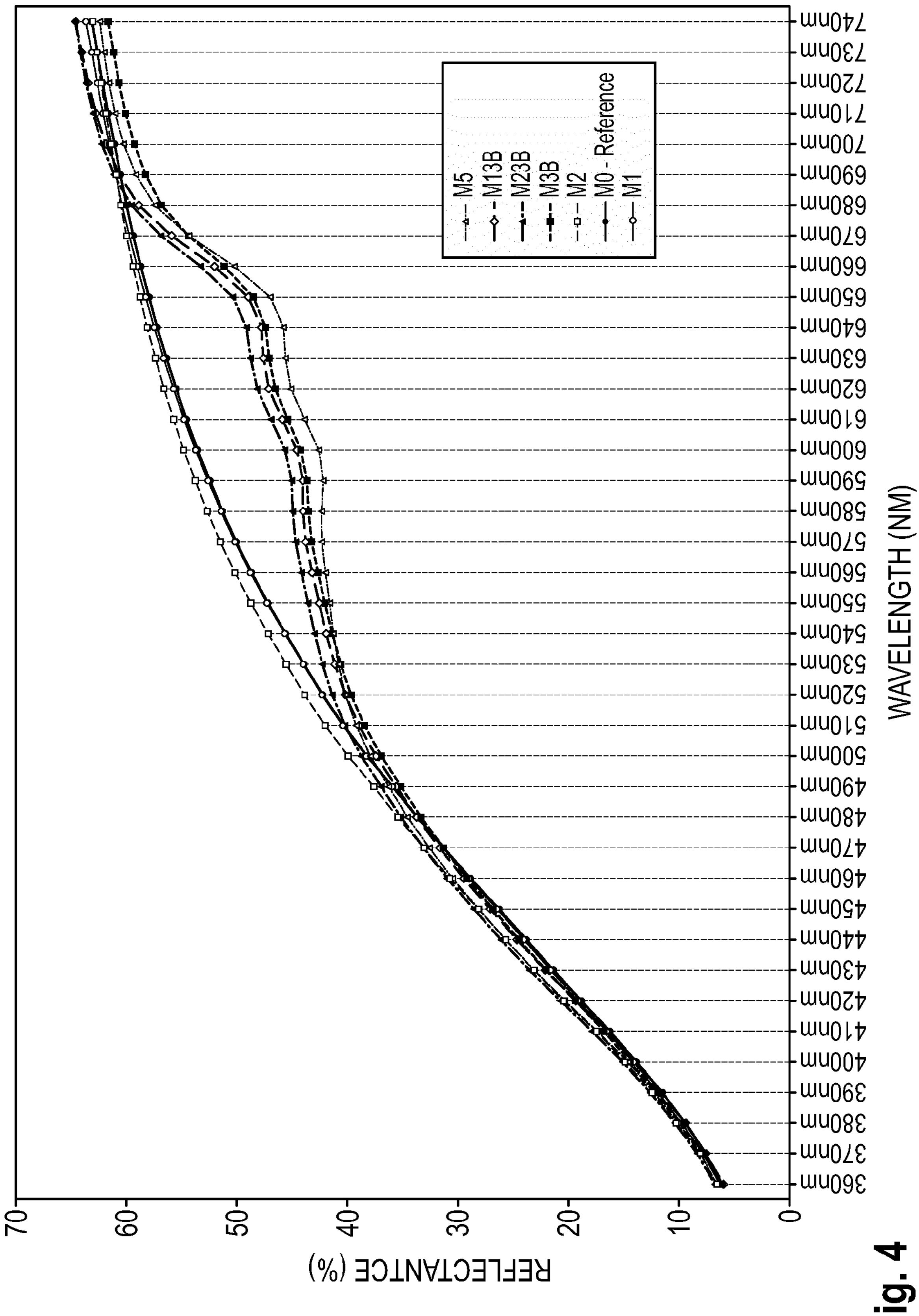

REFLECTANTCE (%)
WAVELENGTH (NM)
0
10
20
30
40
50
60
70
360nm
370nm
380nm
390nm
400nm
410nm
420nm
430nm
440nm
450nm
460nm
470nm
480nm
490nm
500nm
510nm
520nm
530nm
540nm
550nm
560nm
570nm
580nm
590nm
600nm
610nm
620nm
630nm
640nm
650nm
660nm
670nm
680nm
690nm
700nm
710nm
720nm
730nm
740nm
M5
M13B
M23B
M3B
M2
M0 - Reference
M1


Fig. 4

Europäisches Patentamt
European Patent Office
Office européen des brevets

# EUROPEAN SEARCH REPORT

Application Number
EP 16 16 1829

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP H11 269048 A (KAO CORP) 5 October 1999 (1999-10-05) * paragraph [0001] - paragraph [0011] * * examples 1-2 * * paragraph [0026] * | 1-16 | INV. A61K8/35 A61K8/36 A61K8/41 A61K8/42 A61K8/44 A61Q5/06 |
| Y | CA 2 754 965 A1 (PROCTER & GAMBLE [US]) 22 December 2011 (2011-12-22) * paragraph [0013] - paragraph [0014] * * paragraph [0016] * * paragraph [0017] * * claim 10 * | 1-16 | |
| Y | EP 0 313 305 A2 (PROCTER & GAMBLE [US]) 26 April 1989 (1989-04-26) * page 14, line 31 - line 37 * * claims 1-12 * | 1-16 | |
| Y | US 2015/053230 A1 (MYATT GRAHAM JOHN [US]) 26 February 2015 (2015-02-26) * paragraph [0036] - paragraph [0037] * * paragraph [0044] * * paragraph [0045] * * claims 8-9, 12 * | 1-16 | TECHNICAL FIELDS SEARCHED (IPC): A61K A61Q |
| Y | US 2015/053226 A1 (BONAUER CHRISTOPH HANS PETER [DE] ET AL) 26 February 2015 (2015-02-26) * paragraph [0029] * * paragraph [0082] * * paragraph [0079] - paragraph [0080] * | 14,15 | |

The present search report has been drawn up for all claims

2

| Place of search | Date of completion of the search | Examiner |
|---|---|---|
| Munich | 15 June 2016 | Steinheimer, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 16 1829

This annex lists the patent family members relating to the patent documents cited in the above-mentioned European search report. The members are as contained in the European Patent Office EDP file on
The European Patent Office is in no way liable for these particulars which are merely given for the purpose of information.

15-06-2016

| Patent document cited in search report | | Publication date | Patent family member(s) | | | Publication date |
|---|---|---|---|---|---|---|
| JP H11269048 | A | 05-10-1999 | NONE | | | |
| CA 2754965 | A1 | 22-12-2011 | NONE | | | |
| EP 0313305 | A2 | 26-04-1989 | AT | 176396 | T | 15-02-1999 |
| | | | AT | 177939 | T | 15-04-1999 |
| | | | AU | 629518 | B2 | 08-10-1992 |
| | | | AU | 2408588 | A | 27-04-1989 |
| | | | CA | 1334008 | C | 17-01-1995 |
| | | | DE | 3856302 | D1 | 18-03-1999 |
| | | | DE | 3856302 | T2 | 09-09-1999 |
| | | | DE | 3856315 | D1 | 29-04-1999 |
| | | | DE | 3856315 | T2 | 14-10-1999 |
| | | | EP | 0313305 | A2 | 26-04-1989 |
| | | | EP | 0496433 | A2 | 29-07-1992 |
| | | | EP | 0496434 | A2 | 29-07-1992 |
| | | | JP | 2897832 | B2 | 31-05-1999 |
| | | | JP | H01265018 | A | 23-10-1989 |
| | | | NZ | 226667 | A | 25-11-1993 |
| | | | US | 5487884 | A | 30-01-1996 |
| US 2015053230 | A1 | 26-02-2015 | CA | 2919008 | A1 | 26-02-2015 |
| | | | EP | 3036009 | A1 | 29-06-2016 |
| | | | US | 2015053230 | A1 | 26-02-2015 |
| | | | WO | 2015026994 | A1 | 26-02-2015 |
| US 2015053226 | A1 | 26-02-2015 | CA | 2918830 | A1 | 26-02-2015 |
| | | | EP | 2881144 | A2 | 10-06-2015 |
| | | | US | 2015053226 | A1 | 26-02-2015 |
| | | | WO | 2015026992 | A2 | 26-02-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0834303 A2
- US 6849096 B2
- EP 317542 A
- EP 399133 A
- EP 516102 A
- EP 509382 A


### Non-patent literature cited in the description


- **K. KEIS ; K. R. RAMAPRASAD ; Y. K. KAMATH.** Studies of light scattering from ethnic hair fibers. *J. Cosmet. Sci.,* 2004, vol. 55, 49-63